# EUROPEAN PATENT APPLICATION

(11) **EP 3 533 782 A1**
(43) Date of publication of application: **04.09.2019**
(21) Application number: 17865577.5
(22) Date of filing: 30.10.2017
(51) Int. Cl.: C07C 237/20, C07D 207/06, A61K 31/198, A61K 31/40

(54) **FUNCTIONAL DERIVATIVE COMPOUNDS OF ALANINE AND PROLINE AMINO ACIDS AND PHARMACEUTICAL COMPOSITION COMPRISING SAME**

(30) Priority: 28.10.2016 KR 20160142266
(71) Applicant: Amtixbio Co., Ltd., Seoul 05836 (KR)
(72) Inventor: PARK, Ki Duk, Seoul 02792 (KR); BAHN, Yong-Sun, Seoul 03722 (KR); LEE, Jong-Seung, Seoul 01738 (KR); LEE, Kyung-Tae, Seoul 03722 (KR); PAE, Ae Nim, Seoul 02792 (KR); YEON, Seul Ki, Seoul 02792 (KR); KANG, Yong Koo, Seoul 02792 (KR); PARK, Jong Hyun, Seoul 02792 (KR); KIM, Siwon, Seoul 02792 (KR); JANG, Bo Ko, Seoul 02792 (KR); CHOI, Ji Won, Seoul 02792 (KR)
(74) Representative: Bringer IP
(86) International application number: PCT/KR2017/012108
(87) International publication number: WO 2018/080269

(57) **Abstract**

The present invention relates to novel functional derivatives of alanine and proline amino acids. The compounds of the present invention were confirmed to have a very superior antifungal or fungicidal effect. In addition, the compounds of the present invention were confirmed to exhibit a synergistic effect when co-administered with an existing antifungal preparation. Moreover, the compounds of the present invention showed activity on a wide range of fungi. Therefore, the compounds of the present invention can be widely used in a field which requires the treatment with an antifungal or fungicidal preparation against human infectious fungi, animal infectious fungi, and phytopathogenic fungi.

## Description

### Technical Field

The present invention relates to a benzyloxybenzylamine amino acid derivative having an amino acid as a functional group, and an antifungal pharmaceutical composition comprising the same as an active ingredient.

### Background Art

In the past, fungi frequently caused localized infections such as athlete's foot, tinea cruris, and thrush, and rarely caused systemic fungal infections. However, recently, the systemic fungal infection is common enough to represent the fourth most frequent infection among the entire hospital acquired infections. Human fungal infectious diseases can be divided into skin diseases and systemic diseases. Among these, systemic fungal infections are problematic diseases. Over the past two decades, despite the development of medical technology, fungal infections are rather on the rise.

Fungal infectious diseases occur very frequently in patients with decreased bodily immune function due to excessive use of broad-spectrum antibacterial agents, organ transplantation, prolonged administration of anticancer drugs, aging, ADIS, or the like, or in patients with decreased immune function due to procedures such as catheters or prosthetic devices (Beck-Sague, C. M. et al., J. Infect. Dis., 167, 1247-1251, 1993; Diamond, R. D., Rev. Infect. Dis., 13, 480-486, 1991).

In addition, systemic fungal infections are very rapidly increasing to such an extent that about 40% of patients who have been hospitalized due to infections die from fungal diseases. As typical opportunistic pathogens, *Candida albicans*, *Candida glabrata*, *Candida krusei*, *Cryptococcus neoformans,* and the like have been reported (Strenberg, S., Science, 266, 1632-1634, 1994). According to recent studies, it has been reported that fungi are detected in different parts of the brain of patients with Alzheimer's dementia, and thus fungal infections can be a cause of Alzheimer's dementia (Pisa, D. et al. Scientific Reports, 5: 15015).

Meanwhile, *Cryptococcus neoformans* is a basidiomycete fungal pathogen that causes encephalomeningitis in a population with compromised immunity. It is known that 600,000 or more people die annually worldwide due to fungal infectious encephalomeningitis. However, limited therapeutic options are available for treating cryptococcosis

Antifungal agents developed so far can be chemically categorized, largely, into antifungal agents having an azole structure and antifungal agents having a biazole structure. As azole-based antifungal agents, typical drugs include ketoconazole, fluconazole, itraconazole, voriconazole, and the like. As biazole-based antifungal agents, typical drugs include terbinafine, flucytosine, amphotericin B, caspofungin, and the like.

Ketoconazole, fluconazole, itraconazole, and voriconazole which have an azole structure have similar mechanism of action to naftifine and terbinafine which are allylamine-based drugs. Antifungal agents of the above two classes exhibit an action of suppressing enzymes required for a process in which lanosterol is converted to ergosterol, the main component of the fungal cell membrane. The azole-based antifungal agents suppress a microsomal enzyme and the allylamine-based antifungal agents suppress squalene epoxidase, thereby exhibiting the above effect. Flucytosine (5-FC) is a metabolic antagonist that suppresses nucleic acid synthesis, and exhibits an antifungal action by causing miscoding of fungal RNA and antagonizing DNA synthesis in a non-competitive manner. Amphotericin B having a polyene structure exhibits an antifungal action by binding to ergosterol inside the fungal cell membrane so as to induce depolarization of the cell membrane and by forming a hole so as to cause loss of intracellular contents. Caspofungin, an antifungal agent of echinocandin class, has an action of reversibly suppressing fungal cell wall formation, and differs from the above-mentioned antifungal agents that act on the cell membrane in that it acts on the cell wall.

Azole-based drugs may result in hepatitis-induced death when used in patients with decreased hepatic function. Thus, liver function tests must be performed before administration thereof. It has been reported that flucytosine exhibits a myelosuppressive action or hepatotoxicity in a dose-dependent manner, and may cause small bowel colitis. Such side effects are further increased in a case of having decreased renal function. Thus, monitoring of the patient's renal function is very important. In addition, flucytosine is contraindicated in pregnant women. Typical toxicity of amphotericin B is glomerular nephrotoxicity due to renal artery shrinkage which is dose-dependent. Thus, in a case where a lifetime cumulative dose of amphotericin B is 4 g to 5 g or more, the incidence of permanent loss of renal function is increased. In addition, amphotericin B may generate renal toxicity such as excessive loss of potassium, magnesium, and bicarbonate and decreased production of hematopoietic hormones due to tubular toxicity. In addition, amphotericin B may exhibit, as acute reactions, symptoms such as thrombophlebitis, chill, tremor, and hyperventilation. As described above, the previously developed antifungal agents have various side effects depending on the type of drug, and there is a need to develop a new therapeutic agent capable of enhancing an antifungal effect while decreasing such side effects.

### Technical Problem

An object of the present invention is to provide a novel benzyloxybenzylamine amino acid derivative, a salt and/or a solvate thereof.

Another object of the present invention is to provide an antifungal pharmaceutical composition, comprising, as an active ingredient, the benzyloxybenzylamine amino acid derivative of the present invention, a salt and/or a solvate thereof.

Yet another object of the present invention is to provide an antifungal pesticide preparation, comprising, as an active ingredient, the benzyloxybenzylamine amino acid derivative of the present invention, a salt and/or a solvate thereof.

Still yet another object of the present invention is to provide an antifungal preparation for animals, comprising, as an active ingredient, the benzyloxybenzylamine amino acid derivative of the present invention, a salt and/or a solvate thereof.

Still yet another object of the present invention is to provide an antifungal composition, comprising the benzyloxybenzylamine amino acid derivative of the present invention, a salt and/or a solvate thereof.

Still yet another object of the present invention is to provide a human body cleansing composition, a cosmetic composition, or a shampoo composition, comprising the benzyloxybenzylamine amino acid derivative of the present invention, a salt and/or a solvate thereof.

In addition, still yet another object of the present invention is to provide a method for preparing the benzyloxybenzylamine amino acid derivative of the present invention.

### Solution to Problem

In order to achieve the above objects, the present invention provides a benzyloxybenzylamine amino acid derivative represented by the following Formula 1, a pharmaceutically acceptable salt, and/or a solvate thereof.

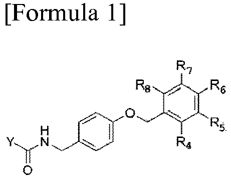

In the Formula 1, R₄, R₅, R₆, R₇, and R₈ are the same or different from one another, and are each independently selected from hydrogen, C₁₋₇ alkyl, hydroxy, halogen, halogenated C₁₋₇ alkyl, C₁₋₇ alkyloxy, and halogenated C₁₋₇ alkyloxy, and Y is selected from the following Formulas 2 to 4: 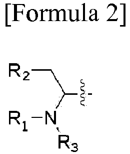 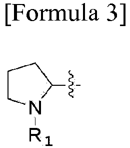 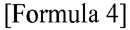 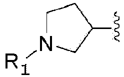 where R₁ and R₃ are the same or different from each other, and are each independently hydrogen or C₁₋₇ alkyl, and
R₂ is C₁₋₇ alkyl.

In the present invention, the halogen may be selected from fluoro, chloro, bromo, and iodo, and the C₁₋₆ alkyl may be a linear, branched, or cyclic alkyl and may be selected from methyl, ethyl, propyl, isopropyl, cyclopropyl, butyl, isobutyl, sec-butyl, t-butyl, pentyl, hexyl, heptyl, and octyl.

The C₁₋₇ alkyloxy group may be selected from methoxy, ethoxy, propoxy, butoxy, pentoxy, hexyloxy, heptyloxy, and octyloxy.

The halogenated C₁₋₇ alkyl may be selected from difluoromethyl, trifluoromethyl, difluoroethyl, trifluoroethyl, trifluoropropyl, trifluoropentyl, trifluorohexyl, and trifluoroheptyl, and the halogenated C₁₋₇ alkyloxy may be selected from difluoromethyloxy, trifluoromethyloxy, difluoroethyloxy, trifluoroethyloxy, trifluoropropyloxy, trifluoropentyloxy, trifluorohexyloxy, and trifluoroheptyloxy.

According to the present invention, in the Formula 1, in a case where any one of R₄, R₅, R₆, R₇, and R₈ is C₁₋₇ alkyl, hydroxy, halogen, halogenated C₁₋₇ alkyl, C₁₋₇ alkyloxy, or halogenated C₁₋₇ alkyloxy, the other four may be hydrogen atoms.

The compound represented by the Formula 1 may be specifically, for example, any one selected from the following compounds, or a racemic mixture of R-form and S-form thereof:
*(R)*/*(S)*-1-((4-((2-(fluorobenzyl)oxy)benzyl)amino)-1-oxobutane-2-aminium chloride (Compound 17);
*(R)*/*(S)*-1-((4-((3-(fluorobenzyl)oxy)benzyl)amino)-1-oxobutane-2-aminium chloride (Compound 18);
*(R)*/*(S)*-1-((4-((4-(fluorobenzyl)oxy)benzyl)amino)-1-oxobutane-2-aminium chloride (Compound 19);
*(R)*/*(S*)-1-oxo-1-((4-((2-(trifluoromethyl)benzyl)oxy)benzyl)amino)butane-2-aminium chloride (Compound 20);
*(R)*/*(S*)-1-oxo-1-((4-((3-(trifluoromethyl)benzyl)oxy)benzyl)amino)butane-2-aminium chloride (Compound 21);
*(R)*/*(S*)-1-oxo-1-((4-((4-(trifluoromethyl)benzyl)oxy)benzyl)amino)butane-2-aminium chloride (Compound 22);
*(R)*/*(S)*-1-((4-((3-(chlorobenzyl)oxy)benzyl)amino)-1-oxobutane-2-aminium chloride (Compound 23);
*(R)*/*(S)*-1-((4-((4-(chlorobenzyl)oxy)benzyl)amino)-1-oxobutane-2-aminium chloride (Compound 24);
*(R)*/*(S)-*1-((4-((3-fluorobenzyl)oxy)benzyl)amino)-1-oxopentane-2-aminium chloride (Compound 25);
*(R)*/*(S)*-1-((4-((4-(trifluorobenzyl)oxy)benzyl)amino)-1-oxopentane-2-aminium chloride (Compound 26);
*(R)*/*(S*)-2-(methylamino)-N-(4-((4-trifluoromethyl)benzyl)oxy)benzyl)butanamide (Compound 27);
*(R)*/*(S*)-N-(4-((3-chlorobenzyl)oxy)benzyl)-2-(methylamino)butanamide (Compound 28);
*(R)*/*(S*)-N-(4-((4-chlorobenzyl)oxy)benzyl)-2-(methylamino)butanamide (Compound 29);
*(R)*/*(S*)-2-(methylamino)-N-(4-((4-trifluoromethyl)benzyl)oxy)benzyl)pentanamide (Compound 30);
*(R)*/*(S*)-2-(methylamino)-N-(4-((4-trifluoromethyl)benzyl)oxy)benzyl)pentanamide (Compound 31);
*(R)*/*(S*)-tert-butyl-2-((4-((3-fluorobenzyl)oxy)benzyl)carbamoyl)pyrrolidine-1-carboxylate (Compound 34);
*(R)*/*(S*)-tert-butyl-2-((4-((4-fluorobenzyl)oxy)benzyl)carbamoyl)pyrrolidine-1-carboxylate (Compound 35);
*(R)*/*(S)-*tert-butyl-2-((4-((4-(trifluoromethyl)benzyl)oxy)benzyl)carbamoyl)pyrrolidine-1-carboxylate (Compound 36);
*(R)*/*(S*)-tert-butyl-2-((4-((4-chlorobenzyl)oxy)benzyl)carbamoyl)pyrrolidine-1-carboxylate (Compound 37);
*(R)*/*(S)-*N-(4-((3-fluorobenzyl)oxy)benzyl)pyrrolidine-2-carboxamide hydrochloride (Compound 38)
*(R)*/*(S*)-N-(4-((4-fluorobenzyl)oxy)benzyl)pyrrolidine-2-carboxamide hydrochloride (Compound 39);
*(R)*/*(S)-*N-(4-((4-(trifluoromethyl)benzyl)oxy)benzyl)pyrrolidine-2-carboxamide hydrochloride (Compound 40);
*(R)*/*(S*)-N-(4-((4-chlorobenzyl)oxy)benzyl)pyrrolidine-2-carboxamide hydrochloride (Compound 41);
*(R)*/*(S*)-1-methyl-N-(4-((3-fluorobenzyl)oxy)benzyl)pyrrolidine-2-carboxamide (Compound 42);
*(R)*/*(S*)-1-methyl-N-(4-((4-(trifluoromethyl)benzyl)oxy)benzyl)pyrrolidine-2-carboxamide (Compound 43);
*(R)*/*(S*)-1-methyl-N-(4-((4-chlorobenzyl)oxy)benzyl)pyrrolidine-2-carboxamide (Compound 44);
*(R)*/*(S*)-tert-butyl-3-((4((3-fluorobenzyl)oxy)benzyl)carbamoyl)pyrrolidine-1-carboxylate (Compound 46);
*(R)*/*(S*)-tert-butyl-3-((4((4-(trifluoromethyl)benzyl)oxy)benzyl)carbamoyl)pyrrolidine-1-carboxylate (Compound 47);
*(R)*/*(S*)-tert-butyl-3-((4((4-chlorobenzyl)oxy)benzyl)carbamoyl)pyrrolidine-1-carboxylate (Compound 48);
*(R)*/*(S*)-2-((4-((3-fluorobenzyl)oxy)benzyl)carbamoyl)pyrrolidi-1-nium chloride (Compound 49);
*(R)*/*(S*)-2-((4-((4-(trifluoromethyl)benzyl)oxy)benzyl)carbamoyl)pyrrolidi-1-nium chloride (Compound 50);
*(R)*/*(S*)-2-((4-((4-chlorobenzyl)oxy)benzyl)carbamoyl)pyrrolidi-1-nium chloride (Compound 51);
*(R)*/*(S*)-1-methyl-N-(4-((3-fluorobenzyl)oxy)benzyl)pyrrolidine-3-carboxamide (Compound 52);
*(R)*/*(S*)*-*1-methyl-N-(4-((4-(trifluoromethyl)benzyl)oxy)benzyl)pyrrolidine-3-carboxamide (Compound 53);
*(R)*/*(S*)-1-methyl-N-(4-((4-chlorobenzyl)oxy)benzyl)pyrrolidine-3-carboxamide (Compound 54);
*(R)*/*(S*)-tert-butyl(1-((4-((3,4-chlorobenzyl)oxy)benzyl)amino)-1-oxobuta-2-nyl)carbamate (Compound 55);
*(R)*/*(S*)-tert-butyl(1-oxo-1-((4-((4-(trifluoromethyl)benzyl)oxy)benzyl)amino)penta-2-nyl)carbamate (Compound 56);
*(R)*/*(S)*-1-((4-((3,4-dichlorobenzyl)oxy)benzyl)amino)-1-oxobutane-2-aminium chloride (Compound 57);
*(R)*/*(S)*-1-((4-((3,4-dichlorobenzyl)oxy)benzyl)amino)-1-oxopentane-2-aminium chloride (Compound 58);
*(R)*/*(S*)-2-(dimethylamino)-N-(4-((4-trifluoromethyl)benzyl)oxy)benzyl)butanamide (Compound 59);
*(R)*/*(S*)-N-(4-((3,4-dichlorobenzyl)oxy)benzyl)-2-(dimethylamino)butanamide (Compound 60);
*(R)*/*(S*)-N-(4-((3,4-dichlorobenzyl)oxy)benzyl)-2-(dimethylamino)butanamide (Compound 61);
*(R)*/*(S*)-2-(ethylamino)-N-(4-((4-(trifluoromethyl)benzyl)oxy)benzyl)butanamide (Compound 62);
*(R)*/*(S*)-N-(4-((3,4-dichlorobenzyl)oxy)benzyl-2-(ethylamino)butanamide (Compound 63);
*(R)*/*(S*)-2-(ethylamino)-N-(4-((4-(trifluoromethyl)benzyl)oxy)benzyl)pentanamide (Compound 64);
*(R)*/*(S*)-N-(4-((3,4-dichlorobenzyl)oxy)benzyl-2-(ethylamino)pentanamide (Compound 65);
*(R)*/*(S*)-2-(diethylamino)-N-(4-((4-(trifluoromethyl)benzyl)oxy)benzyl)butanamide (Compound 66);
*(R)*/*(S*)-N-(4-((3,4-dichlorobenzyl)oxy)benzyl-2-(diethylamino)butanamide (Compound 67);
*(R)*/*(S*)-2-(diethylamino)-N-(4-((4-(trifluoromethyl)benzyl)oxy)benzyl)pentanamide (Compound 68);
*(R)*/*(S*)-N-(4-((3,4-dichlorobenzyl)oxy)benzyl-2-(diethylamino)pentanamide (Compound 69).

In the present invention, the pharmaceutically acceptable salt is not particularly limited as long as it is conventionally used in the art. Specific examples thereof include, but are not limited to, those capable of forming a salt with an inorganic acid such as hydrochloric acid, bromic acid, sulfonic acid, amidosulfuric acid, phosphoric acid or nitric acid, or an organic acid such as carboxylic acid or sulfonic acid.

In addition, examples of the carboxylic acid may include acetic acid, maleic acid, fumaric acid, malic acid, citric acid, tartaric acid, lactic acid, benzoic acid, succinic acid, propionic acid, glycolic acid, stearic acid, lactic acid, and the like, and examples of the sulfonic acid may include methansulfonic acid, ethanesulfonic acid, benzenesulfonic acid, toluenesulfonic acid, and naphthalenedisulfonic acid. However, types of the carboxylic acid and sulfonic acid are not limited to the above-mentioned compounds.

The benzyloxybenzylamine amino acid derivative represented by the Formula 1 according to the present invention effectively acts to suppress growth of, or kill fungi. Therefore, a composition comprising, as an active ingredient, the benzyloxybenzylamine amino acid derivative represented by the Formula 1 can be usefully used as a pharmaceutical composition for preventing or treating mycosis. In addition, the benzyloxybenzylamine amino acid derivative can be prepared as a pharmaceutical composition for administration in combination with existing drugs, and used.

In the present invention, the mycosis is not limited as long as the mycosis is a disease caused by fungi. Examples of the fungi include, but are not limited to, Candida *spp.*, Sporotrichum *spp.*, Malassezia *spp.*, Cryptococcus *spp.*, Aspergillus *spp.*, Pneumocystis jirovecii *spp.*, Mucor *spp.*, and the like.

In the present invention, the mycosis may be a disease caused by Candida *spp.*, Sporotrichum *spp.*, Malassezia *spp.*, Cryptococcus *spp.*, Aspergillus *spp.*, Pneumocystis jirovecii *spp.*, or Mucor *spp.* Specific examples thereof include, but are not limited to, encephalomeningitis, skin candidiasis, mucosal candidiasis, visceral candidiasis, urinary candidiasis, candida endocarditis, oropharyngeal candidiasis, candida endophthalmitis, candida septicemia, trichophytosis, tinea versicolor, sporotrichosis, fungal abscess, fungal granuloma, granuloma pyogenicum, maduromycosis, pneumocystis jirovecii pneumonia, systemic cryptococcosis, skin mucosal cryptococcosis, aspergillosis, cavity, hemoptysis, allergy, old pulmonary tuberculosis, pulmonary fibrosis, pulmonary cyst, chronic fever, cough, sputum, blood-stained sputum, and zygomycosis.

The benzyloxybenzylamine amino acid derivative according to the present invention can be prepared by a method comprising the following steps of:
1) protecting an amino group of an amino acid selected from the following Formulas 5 to 7 with a protecting group;
2) reacting the amino acid having a protected amine group with a benzylamine derivative, to prepare a benzyloxybenzylamine amino acid derivative in which the amino group of the amino acid is protected with the protecting group; and
3) removing the amine protecting group.

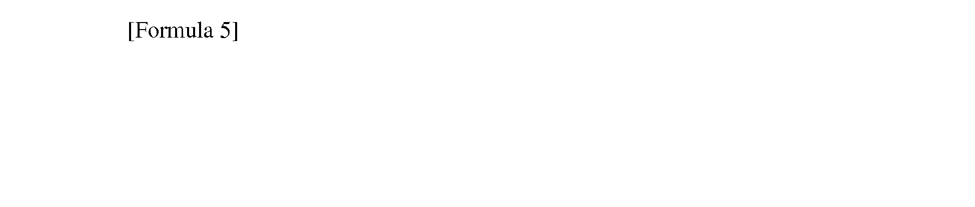

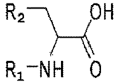

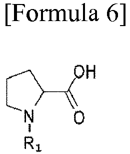

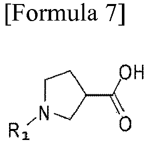

In the Formulas 5 to 7,
R₁ is hydrogen or C₁₋₇ alkyl,
R₂ is C₁₋₇ alkyl.

First, the amine group of the amino acid selected from the Formulas 5 to 7 is protected with an amine protecting group. For the amine protecting group, any group may be used as long as it is conventionally used for amine protection.

Next, the amino acid having a protected amine group is reacted with a benzylamine derivative to prepare a benzyloxybenzylamine amino acid derivative in which the amine group of the amino acid is protected with the protecting group. The reaction may be performed in one step or in two steps. In a case where the reaction is performed in one step, the amino acid having a protected amine group may be reacted with a benzyloxybenzylamine derivative. In a case where the reaction is performed in two steps, the amino acid having a protected amine group may be reacted with 4-hydroxybenzylamine to prepare a 4-hydroxybenzylamine amino acid derivative in which the amine group of the amino acid is protected with the protecting group, and then the resultant may be reacted with a benzyl bromide derivative.

The amine protecting group may be removed using a conventional amine removal method. In the present invention, treatment with perchloric acid was used.

In the benzyloxybenzylamine amino acid derivative according to the present invention, the amine of the amino acid residue may be primary, secondary, or tertiary. In a case where the amine is secondary or tertiary, an alkylation reaction may be performed to introduce an alkyl group.

According to the present invention, the benzyloxybenzylamine amino acid derivative or a pharmaceutically acceptable salt thereof may be formulated with a conventional formulation method by being mixed with a conventional carrier, adjuvant, or diluent, and may be prepared in a form suitable for oral administration or parenteral administration.

Examples of the carrier, adjuvant, or diluent may include lactose, dextrose, sucrose, sorbitol, mannitol, xylitol, erythritol, maltitol, starch, alginate, gelatin, calcium phosphate, calcium silicate, cellulose, methylcellulose, microcrystalline cellulose, polyvinyl pyrrolidone, water, methylhydroxybenzoate, propylhydroxybenzoate, talc, magnesium stearate, and mineral oil.

In addition, in a case of being made into a preparation, conventional fillers, extenders, binders, wetting agents, disintegrants, surfactants, and the like may be further contained, and lubricants such as magnesium stearate and talc may be further added.

For the oral administration, the formulation may be prepared in the form of tablets, capsules, solutions, syrups, suspensions, or the like. For the parenteral administration, the formulation may be prepared in the form of intraperitoneal, subcutaneous, intramuscular or transdermal injections, or external preparations.

According to an embodiment of the present invention, the pharmaceutical composition inhibits growth of, or kills fungi. The effective daily dose of the benzyloxybenzylamine amino acid derivative of Formula 1 or a pharmaceutically acceptable salt thereof in the pharmaceutical composition is 0.01 to 1,000 mg/day on an adult basis. However, the dose may vary depending on the patient's age, body weight, sex, dosage form, health condition and severity of disease, and may be administered once a day or divided into several times a day at a certain time interval according to the judgment of a doctor or a pharmacist.

### Advantageous Effects of Invention

The benzyloxybenzylaminyl amino acid derivative according to the present invention exhibits excellent antifungal activity while exhibiting excellent drug properties in the assessment by ADME/Tox testing, and has no or greatly alleviated side effects seen in existing antifungal agents such as hepatotoxicity, nephrotoxicity, neurotoxicity, rash, edema, and bone marrow suppression. Thus, the benzyloxybenzylaminyl amino acid derivative can be usefully applied as a new therapeutic agent for fungal infectious diseases.

### Brief Description of Drawings

FIG. 1 illustrates a graph obtained by comparing suppressive activity of the compound of the present invention with that of amphotericin B (AmpB) and fluconazole (FCZ), against strains *Candida albicans* SC5314 (ATCC), *Candida glabrata* BG2 (ATCC), *Cryptococcus neoformans var. grubii* H99 (ATCC), and *Aspergillus fumigatus.* In FIG. 1, KDS1087 represents Compound 22, KDS1090 represents Compound 26, and KDS1092 represents Compound 29. In the drawing, from the left side of the bar graph, KDS1087, KDS1090, KDS1092, AmpB, and FCZ appear in that order.
FIG. 2 illustrates a graph obtained by comparing MIC test results of Compound 22 (1087), Compound 26 (1090), and Compound 28 (1092) of the present invention with that of amphotericin B (AMB), against several types of *Aspergillus fumigatus* strains (average values are indicated). The compound of the present invention exhibited a higher inhibitory activity than amphotericin B. In the drawing, from the left side of the bar graph, KDS1087, KDS1090, KDS1092, and AmpB appear in that order.
FIG. 3 illustrates average values of MIC test results of Compound 22 (1087), Compound 26 (1090), and Compound 28 (1092) of the present invention, against several types of *Candida albicans* strains. In the drawing, from the left side of the bar graph, KDS1087, KDS1090, and KDS1092 appear in that order.
FIG. 4 illustrates results obtained by identifying very excellent suppressive activity of the compound (Compound 26) of the present invention against phytopathogenic fungi as a result of applying the compound thereto.
FIG. 5 illustrates results obtained by comparing suppressive activity of the compound of the present invention with that of propiconazole, difenoconazole, and fludioxonil, against various pathogenic fungi.
FIG. 6 illustrates results obtained by assessing drug efficacy of the compound of the present invention in a *Galleria mellonella* insect model.

### Detailed Description of Invention

Schemes for preparing the compounds of the present invention are as follows. In the following schemes, numbers which are not used together with alphabetic letters represent compound numbers prepared in the present invention. That is, 1 represents Compound 1:

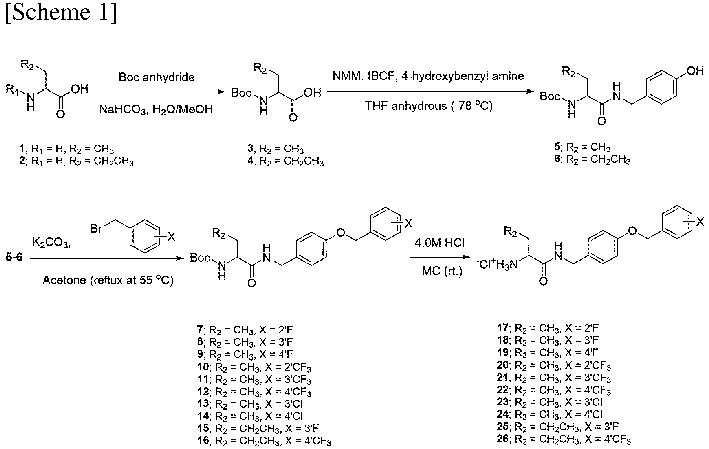

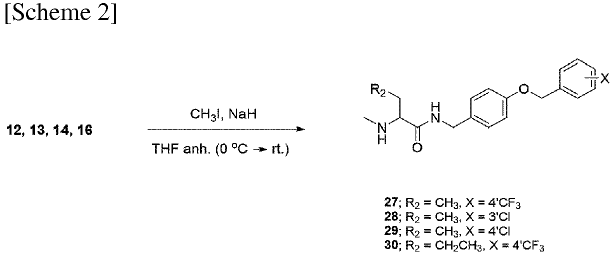

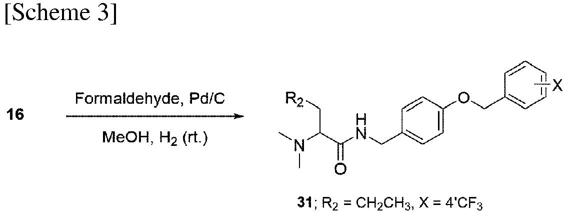

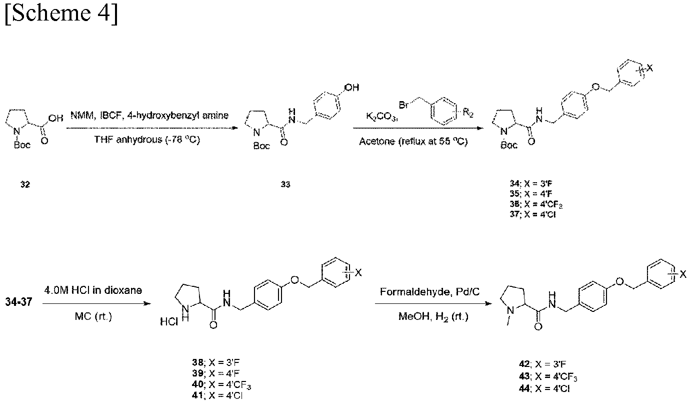

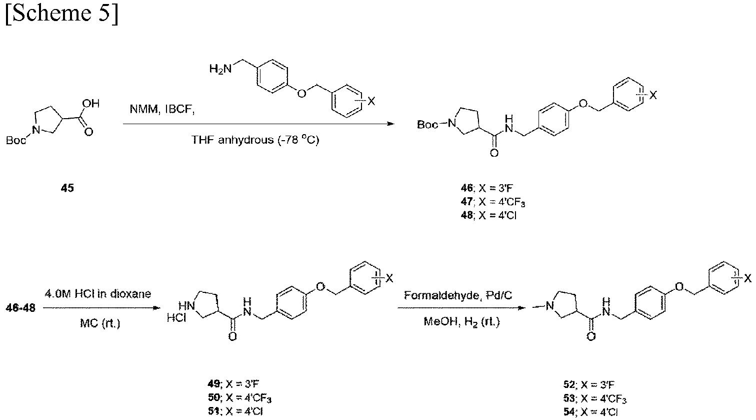

Synthesis examples for preparing the compounds of the present invention are as follows:

### Synthesis Example 1: Protection of amine group of amino acid

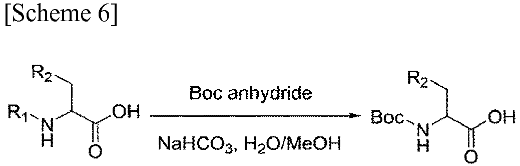

An amino acid (1.0 eq.), Boc anhydride (1.5 eq.), and sodium bicarbonate (1.5 eq.) were dissolved in a mixed solvent of distilled water and methanol (1:1), and allowed to react at room temperature for 36 to 48 hours. After completion of the reaction, the resultant was washed twice with diethyl ether, and then the pH of the water layer was adjusted to 2 with 1.0 M hydrochloric acid. Then, extraction with ethyl acetate (EA) was performed. The obtained organic layer was dehydrated using a desiccant (sodium sulfate) and dried under reduced pressure, to obtain the desired compound, an amino acid of which an amine group had been protected with a protecting group (Boc).

### Synthesis Example 2: Binding with 4-hydroxybenzylamine

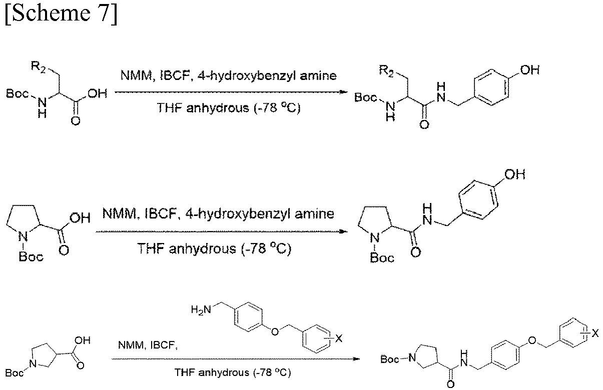

The compound (0.1 M) synthesized in the Synthesis Example 1 and N-methylmorpholine (NMM) (0.15 M, 1.5 eq.) were added to anhydrous tetrahydrofuran at a low temperature of -78°C, and the mixture was stirred for 5 minutes. Then, isobutyl chloroformate (IBCF) (0.13 M, 1.3 eq.) was added thereto, and the resultant was stirred for 5 minutes. Then, 4-hydroxybenzylamine derivative (0.12 M, 1.2 eq.) was added thereto and the resultant was stirred for 5 minutes. The temperature of the reaction mixture was raised to room temperature, and then it was identified whether the reaction proceeded while performing stirring for 30 minutes to 60 minutes. After completion of the reaction, the mixture was filtered and dried under reduced pressure, and then purified by silica gel column chromatography.

### Synthesis Example 3: Reaction for binding with benzyl bromide

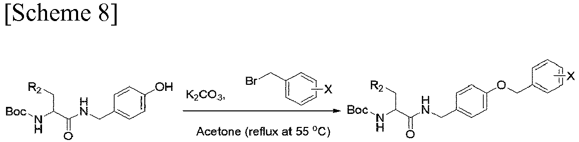

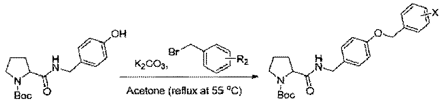

The compound (1.0 eq.) of the Synthesis Example 2, benzyl bromide (1.0 to 1.2 eq.), and potassium carbonate (4.0 eq.) were dissolved in acetone, and then it was identified whether the reaction proceeded while performing refluxing at 50°C to 60°C for 4 to 12 hours. After completion of the reaction, the reaction mixture was concentrated. Then, extraction with methylene chloride was performed, and water remaining in the organic layer was removed with a desiccant (sodium sulfate). The obtained organic layer was concentrated and then purified by silica gel column chromatography.

### Synthesis Example 4: Removal of amine protecting group

The compound obtained in the Synthesis Example 3 was dissolved in methylene chloride. Then, an excessive amount (6 to 10 eq.) of 4.0 M hydrochloric acid was added thereto and stirring was performed at room temperature. After completion of the reaction, the resulting solid was washed with ethyl acetate and then dried under vacuum to remove the amine protecting group (boc). As a result, a benzyloxybenzylamine amino acid derivative containing the desired primary amine structure was obtained.

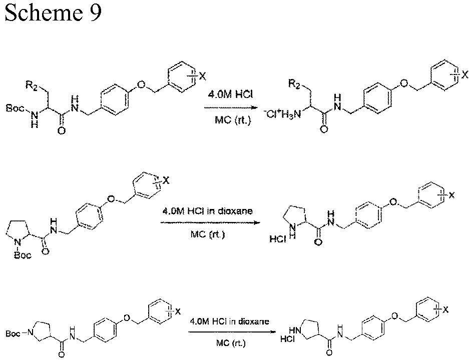

### Synthesis Example 5.1: Alkylation 1

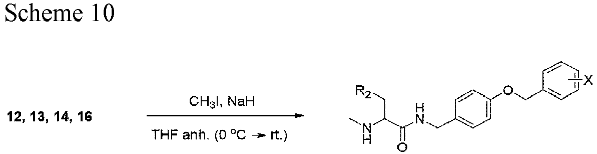

The benzyloxybenzylamine amino acid derivative of which the amino acid has a primary amine structure was subjected to alkylation, to have a secondary amine structure. Specifically, the compound (1.0 eq.) of Synthesis Example 4 and iodomethane (10 eq.) were dissolved in tetrahydrofuran, and then sodium hydride (10 eq.) was slowly added dropwise thereto at 0°C. Then, the resultant was allowed to react at room temperature for 24 hours. After completion of the reaction, the reaction mixture was extracted with methylene chloride and dehydrated with a desiccant (sodium sulfate). Then, concentration under reduced pressure was performed. The obtained concentrate was purified by silica gel column chromatography. The purified compound was dissolved in methylene chloride and then the resultant was stirred while adding 6.0 to 10.0 eq. of 4.0 M hydrochloric acid thereto. After completion of the reaction, the resultant was concentrated under reduced pressure, and then the concentrate was purified by silica gel column chromatography to obtain a benzyloxybenzylamine amino acid derivative containing a secondary amine structure.

### Synthesis Example 5.2: Alkylation 2

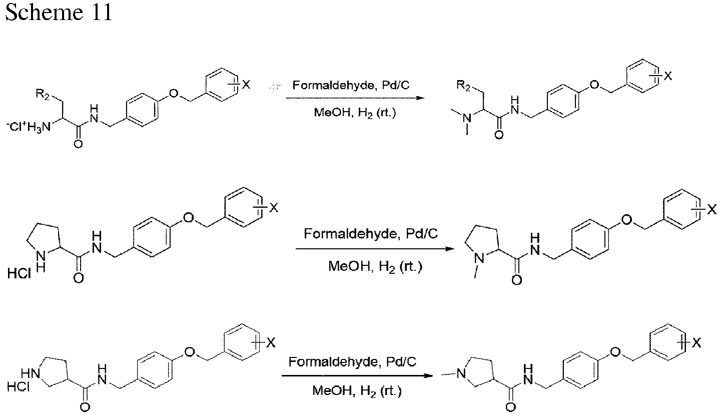

The benzyloxybenzylamine amino acid derivative of which the amino acid has a primary amine structure was subjected to alkylation, to have a tertiary amine structure. Specifically, the compound (1.0 eq.) of Synthesis Example 4 was dissolved in methanol. Then, formaldehyde (37% by weight solution) and 10% palladium catalyst were sequentially added thereto, and the resultant was allowed to react at room temperature for 18 hours. After the reaction, the catalyst was filtered off with celite, and the filtrate was evaporated under reduced pressure. The reaction mixture was recrystallized from methanol/diethyl ether to obtain the purified desired compound.

### Synthesis Example 6: General method for ethylation and diethylation of amine

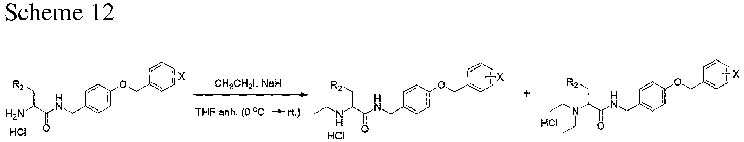

The compound (1.0 eq.) obtained in the Synthesis Example 4 and iodoethane (0.9 eq.) were dissolved in a tetrahydrofuran solvent, and sodium hydride (10 eq.) was added dropwise very slowly thereto at 0°C. The resultant was allowed to react at room temperature for 24 hours. After completion of the reaction, the resultant was diluted with a dimethyl chloride solvent and washed with distilled water. Then, water in the organic layer was dried over sodium sulfate and concentrated *in vacuo.* The obtained residue was separated and purified by chromatography using silica gel. The purified product was dissolved in a dimethyl chloride solvent, and then 4.0M hydrochloric acid (6.0 to 10.0 eq.) was added thereto while performing stirring at room temperature. Then, concentration *in vacuo* was performed. The obtained residue was separated and purified by chromatography using silica gel.

Hereinafter, the present invention will be described in more detail with reference to preferred examples. However, it will be apparent to those skilled in the art that these examples are intended to more specifically describe the present invention and the scope of the present invention is not limited thereby.

### Preparation examples

### (R)/(S)-2-((tert-butoxycarbonyl)amino)butanoic acid (Compound 3)

Using the above Scheme 6, Compound 1 (3.00 g, 29.1 mmol), Boc anhydride (10.00 mL, 43.6 mmol), and NaHCO₃ (3.67 g, 43.6 mmol) were reacted to synthesize Compound 3 in the form of white powder (5.89 g, 100%): R_{f} = 0.50 (DCM 19 : Methanol 1 and few drops of acetic acid); ¹H NMR (DMSO-d₆, 400 MHz) 12.40 (C(O)OH), 7.02 (d, J = 7.9 Hz, NH), 3.74-3.82 (m, NHCHCH₂), 1.50-1.73 (m, CH₂CH₃), 1.38 (s, Boc), 0.87 (t, J = 7.4 Hz, CH₂CH₃).

### (R)/(S)-2-((tert-butoxycarbonyl)amino)pentanoic acid (Compound 4)

Using the above Scheme 6, Compound 2 (3.00 g, 25.6 mmol), Boc anhydride (8.82 mL, 38.4 mmol), and NaHCO₃ (3.24 g, 38.4 mmol) were reacted to synthesize Compound 4 in the form of white powder (5.07 g, 91%): R_{f} = 0.50 (DCM 19 : Methanol 1 and few drops of acetic acid); ¹H NMR (DMSO-d₆, 400 MHz) 12.38 (C(O)OH), 7.03 (d, J = 8.0 Hz, NH), 3.81-3.89 (m, NHCHCH₂), 1.47-1.64 (m, CH₂CH₃), 1.38 (s, Boc), 0.85 (t, J = 7.4 Hz, CH₂CH₃).

### (R)/(S)-tert-butyl(1-((4-hydroxybenzyl)amino)-1-oxobuta-2-nyl)carbamate (Compound 5)

Using the above Scheme 7, Compound 3 (1.05 g, 5.2 mmol), NMM (0.85 mL, 7.8 mmol), IBCF (0.85 mL, 6.6 mmol), and 4-hydroxybenzylamine (0.76 g, 6.2 mmol) were reacted to synthesize Compound 5 in the form of light pink powder was synthesized (1.27 g, 80%): R_{f} = 0.35 (EtOAc 1 : n-hexane 1); ¹H NMR (DMSO-d₆, 400 MHz) 9.27 (s, Ar-OH), 8.15 (t, J = 5.7 Hz, C(O)NH), 7.03 (d, J = 8.1 Hz, ArH), 6.67 (d, J = 8.0 Hz, ArH), 6.78 (d, J = 8.0 Hz, Boc-NH), 4.08-4.22 (m, NHCH₂), 3.79-3.87 (m, NHCHCH₂), 1.45-1.67 (m, CH₂CH₃), 1.38 (s, Boc), 0.82 (t, J = 7.3 Hz, CH₂CH₃). *(R)*/*(S*)-tert-butyl(1-((4-hydroxybenzyl)amino)-1-oxopenta-2-nyl)carbamate (Compound 6)

Using the above Scheme 7, Compound 4 (4.50 g, 20.7 mmol), NMM (3.41 mL, 31.1 mmol), IBCF (3.41 mL, 26.3 mmol), and 4-hydroxybenzylamine (3.06 g, 24.9 mmol) were reacted to synthesize Compound 6 in the form of light pink power (4.09 g, 61%): R_{f} = 0.45 (EtOAc 1 : n-hexane 1); ¹H NMR (DMSO-d₆, 400 MHz) 9.27 (s, Ar-OH), 8.15 (t, J = 5.7 Hz, Boc-NH), 7.03 (d, J = 8.1 Hz, ArH), 6.67 (d, J = 8.0 Hz, ArH), 6.78 (d, J = 8.0 Hz, C(O)NH), 4.08-4.22 (m, NHCH₂), 3.79-3.87 (m, NHCHCH₂), 1.45-1.67 (m, CH₂CH₃), 1.38 (s, Boc), 0.82 (t, J = 7.3 Hz, CH₂CH₃).

### (R)/(S)-tert-butyl(1-((4-((2-fluorobenzyl)oxy)benzyl)amino)-1-oxobuta-2-nyl)carbamate (Compound 7)

Using the above Scheme 8, Compound 5 (0.10 g, 0.3 mmol), 2-fluorobenzyl bromide (0.07 g, 0.4 mmol), and K₂CO₃ (0.18 g, 1.3 mmol) were reacted to synthesize 7 in the form of white powder (0.11 g, 81%): R_{f} = 0.60 (EtOAc 1 : n-hexane 1); ¹H NMR (DMSO-d₆, 400 MHz) 8.22 (t, J = 5.9 Hz, C(O)NH), 7.50-7.57 (m, ArH), 7.38-7.45 (m, ArH), 7.15-7.28 (m, ArH), 6.93-6.98 (m, ArH), 6.78-6.83 (m, Boc-NH), 5.11 (s, OCH₂), 4.14-4.27 (m, NHCH₂), 3.80-3.87 (m, NHCHCH₂), 1.46-1.67 (m, CH₂CH₃), 1.38 (s, Boc), 0.82 (t, J = 7.4 Hz, CH₂CH₃).

### (R)/(S)-tert-butyl(1-((4-((3-fluorobenzyl)oxy)benzyl)amino)-1-oxobuta-2-nyl)carbamate (Compound 8)

Using the above Scheme 8, Compound 5 (0.20 g, 1.0 mmol), 3-fluorobenzyl bromide (0.27 g, 1.2 mmol), and K₂CO₃ (0.55 g, 4.0 mmol) were reacted to synthesize 8 in the forme of white power (0.16 g, 39%): R_{f} = 0.50 (EtOAc 1 : n-hexane 1); ¹H NMR (DMSO-d₆, 400 MHz) ¹H NMR (DMSO-d₆, 400 MHz) 8.21 (t, J = 5.8 Hz, C(O)NH), 7.39-7.47 (m, ArH), 7.23-7.29 (m, ArH), 7.11-7.20 (m, ArH), 6.91-6.97 (m, ArH), 6.80 (d, J = 8.0 Hz, Boc-NH), 5.11 (s, OCH₂), 4.13-4.27 (m, NHCH₂), 3.80-3.87 (m, NHCHCH₂), 1.45-1.67 (m, CH₂CH₃), 1.37 (s, Boc), 0.82 (t, J = 7.3 Hz, CH₂CH₃).

### (R)/(S)-tert-butyl(1-((4-((4-fluorobenzyl)oxy)benzyl)amino)-1-oxobuta-2-nyl)carbamate (Compound 9)

Using the above Scheme 8, Compound 5 (0.20 g, 0.7 mmol), 4-fluorobenzyl bromide (0.09 g, 0.7 mmol), and K₂CO₃ (0.36 g, 2.6 mmol) were reacted to synthesize 9 in the form of white powder (0.24 g, 89%): R_{f} = 0.63 (EtOAc 1 : n-hexane 1); ¹H NMR (DMSO-d₆, 400 MHz) ¹H NMR (DMSO-d₆, 400 MHz) 8.21 (t, J = 4.7 Hz, C(O)NH), 7.44-7.53 (m, ArH), 7.13-7.26 (m, ArH), 6.90-6.97 (m, ArH), 6.80 (d, J = 7.7 Hz, Boc-NH), 5.06 (s, OCH₂), 4.13-4.28 (m, NHCH₂), 3.79-3.89 (m, NHCHCH₂), 1.45-1.69 (m, CH₂CH₃), 1.37 (s, Boc), 0.82 (t, J = 7.0 Hz, CH₂CH₃).

### (R)/(S)-tert-butyl(1-oxo-1-((4-((2-(trifluoromethyl)benzyl)oxy)benzyl)amino)buta-2-nyl)carbamate (Compound 10)

Using the above Scheme 8, Compound 5 (0.10 g, 0.3 mmol), 2-(trifluoromethyl)benzyl bromide (0.08 g, 0.4 mmol), and K₂CO₃ (0.18 g, 1.3 mmol) were reacted to synthesize 10 in the form of white powder (0.13 g, 89%): R_{f} = 0.63 (EtOAc 1 : n-hexane 1); ¹H NMR (DMSO-d₆, 400 MHz) 8.22 (t, J = 5.7 Hz, C(O)NH), 7.68-7.83 (m, ArH), 7.55-7.62 (m, ArH), 7.16-7.23 (m, ArH), 6.91-6.97 (m, ArH), 6.80 (d, J = 7.9 Hz, Boc-NH), 5.21 (s, OCH₂), 4.14-4.29 (m, NHCH₂), 3.80-3.88 (m, NHCHCH₂), 1.47-1.69 (m, CH₂CH₃), 1.37 (s, Boc), 0.82 (t, J = 7.2 Hz, CH₂CH₃).

### (R)/(S)-tert-butyl(1-oxo-1-((4-((3-(trifluoromethyl)benzyl)oxy)benzyl)amino)buta-2-nyl)carbamate (Compound 11)

Using the above Scheme 8, Compound 5 (0.30 g, 1.0 mmol), 3-(trifluoromethyl)benzyl bromide (0.17 mL, 1.1 mmol), and K₂CO₃ (0.54 g, 3.9 mmol) were reacted to synthesize 11 in the form of white powder (0.20 g, 44%): R_{f} = 0.54 (EtOAc 1 : n-hexane 1) ¹H NMR (DMSO-d₆, 400 MHz) 8.22 (t, J = 5.7 Hz, C(O)NH), 7.57-7.82 (m, ArH), 7.11-7.21 (m, ArH), 6.90-6.99 (m, ArH), 6.80 (d, J = 8.1 Hz, Boc-NH), 5.20 (s, OCH₂), 4.14-4.28 (m, NHCH₂), 3.78-3.88 (m, NHCHCH₂), 1.44-1.68 (m, CH₂CH₃), 1.37 (s, Boc), 0.82 (t, J = 7.2 Hz, CH₂CH₃).

### (R)/(S)-tert-butyl(1-oxo-1-((4-((4-(trifluoromethyl)benzyl)oxy)benzyl)amino)propa-2-nyl)carbamate (Compound 12)

Using the above Scheme 8, Compound 5 (0.30 g, 1.0 mmol), 4-(trifluoromethyl)benzylbromide (0.17 mL, 1.1 mmol), and K₂CO₃ (0.54 g, 3.9 mmol) were reacted to synthesize 12 in the form of white powder (0.44 g, 96%): R_{f} = 0.24 (EtOAc 1 : n-hexane 1); ¹H NMR (DMSO-d₆, 400 MHz) 8.21 (t, J = 5.8 Hz, C(O)NH), 7.62-7.80 (m, ArH), 7.14-7.21 (m, ArH), 6.91-6.98 (m, ArH), 6.80 (d, J = 7.9 Hz, Boc-NH), 5.21 (s, OCH₂), 4.13-4.28 (m, NHCH₂), 3.80-3.87 (m, NHCHCH₂), 1.46-1.67 (m, CH₂CH₃), 1.37 (s, Boc), 0.82 (t, J = 7.3 Hz, CH₂CH₃).

### (R)/(S)-tert-butyl(1-((4-((3-chlorobenzyl)oxy)benzyl)amino)-1-oxobuta-2-nyl)carbamate (Compound 13)

Using the above Scheme 8, Compound 5 (0.30 g, 1.0 mmol), 3-chlorobenzyl bromide (0.22 g, 1.1 mmol), and K₂CO₃ (0.54 g, 3.9 mmol) were reacted to synthesize 13 in the form of white powder (0.38 g, 89%): R_{f} = 0.54 (EtOAc 1 : n-hexane 1); ¹H NMR (DMSO-d₆, 400 MHz) 8.21 (t, J = 5.9 Hz, C(O)NH), 7.48-7.51 (m, ArH), 7.35-7.45 (m, ArH), 7.15-7.19 (m, ArH), 6.91-6.96 (m, ArH), 6.80 (d, J = 8.1 Hz, Boc-NH), 5.21 (s, OCH₂), 4.13-4.27 (m, NHCH₂), 3.80-3.88 (m, NHCHCH₂), 1.45-1.67 (m, CH₂CH₃), 1.37 (s, Boc), 0.82 (t, J = 7.4 Hz, CH₂CH₃).

### (R)/(S)-tert-butyl(1-((4-(4-chlorobenzyl)oxy)benzyl)amino)-1-oxobuta-2-nyl)carbamate (Compound 14)

Using the above Scheme 8, Compound 5 (0.20 g, 0.7 mmol), 4-chlorobenzyl bromide (0.09 mL, 0.7 mmol), and K₂CO₃ (0.36 g, 2.6 mmol) were reacted to synthesize a compound in the form of white powder (0.28 g, 100%): R_{f} = 0.50 (EtOAc 1 : n-hexane 1); ¹H NMR (DMSO-d₆, 400 MHz) 8.21 (t, J = 5.9 Hz, C(O)NH), 7.42-7.52 (m, ArH), 7.13-7.20 (m, ArH), 6.90-6.95 (m, ArH), 6.80 (d, J = 8.0 Hz, Boc-NH), 5.08 (s, OCH₂), 4.13-4.27 (m, NHCH₂), 3.79-3.87 (m, NHCHCH₂), 1.45-1.68 (m, CH₂CH₃), 1.37 (s, Boc), 0.82 (t, J = 7.3 Hz, CH₂CH₃).

### (R)/(S)-tert-butyl(1-((4-(3-fluorobenzyl)oxy)benzyl)amino)-1-oxopenta-2-nyl)carbamate (Compound 15)

Using the above Scheme 8, Compound 6 (0.50 g, 1.6 mmol), 3-fluorobenzyl bromide (0.31 g, 1.6 mmol), and K₂CO₃ (0.86 g, 6.2 mmol) were reacted to synthesize a compound in the form of white powder (0.62 g, 92%): R_{f} = 0.33 (EtOAc 1 : n-hexane 3); ¹H NMR (DMSO-d₆, 400 MHz) 8.20 (t, J = 5.8 Hz, C(O)NH), 7.39-7.46 (m, ArH), 7.23-7.29 (m, ArH), 7.11-7.18 (m, ArH), 6.91-6.96 (m, ArH), 6.82 (d, J = 8.0 Hz, Boc-NH), 5.11 (s, OCH₂), 4.13-4.25 (m, NHCH₂), 3.86-3.94 (m, NHCHCH₂), 1.41-1.60 (m, CH₂CH₂CH₃), 1.37 (s, Boc), 1.20-1.32 (m, CH₂CH₂CH₃), 0.84 (t, J = 7.3 Hz, CH₂CH₂CH₃).

### (R)/(S)-tert-butyl(1-oxo-1-((4-((4-(trifluoromethyl)benzyl)oxy)benzyl)amino)penta-2-nyl)carbamate (Compound 16)

Using the above Scheme 8, Compound 6 (0.50 g, 1.6 mmol), 4-(trifluoromethyl)benzyl bromide (0.39 g, 1.6 mmol), and K₂CO₃ (0.86 g, 6.2 mmol) were reacted to synthesize a compound in the form of white powder (0.70 g, 94%): R_{f} = 0.37 (EtOAc 1 : n-hexane 3); ¹H NMR (DMSO-d₆, 400 MHz) 8.20 (t, J = 5.9 Hz, C(O)NH), 7.63-7.78 (m, ArH), 7.13-7.19 (m, ArH), 6.92-6.97 (m, ArH), 6.82 (d, J = 8.2 Hz, Boc-NH), 5.21 (s, OCH₂), 4.13-4.25 (m, NHCH₂), 3.86-3.94 (m, NHCHCH₂), 1.41-1.60 (m, CH₂CH₂CH₃), 1.37 (s, Boc), 1.18-1.32 (m, CH₂CH₂CH₃), 0.84 (t, J = 7.3 Hz, CH₂CH₂CH₃).

### (R)/(S)-1-((4-((2-(fluorobenzyl)oxy)benzyl)amino)-1-oxobutane-2-aminum chloride (Compound 17)

Using the above Scheme 9, Compound 7 (0.09 g, 0.22 mmol) and 4.0 M HCl in dioxane (0.38 mL, 10.8 mmol) were reacted to synthesize Compound 17 in the form of white powder (0.02 g, 22%): R_{f} = 0.00 (EtOAc 9 : Acetone 1); ¹H NMR (DMSO-d₆, 400 MHz) 8.85 (t, J = 5.3 Hz, C(O)NH), 8.06 (broad, NH₃⁺Cl⁻), 7.38-7.57 (m, ArH), 7.20-7.29 (m, ArH), 6.97-7.02 (m, ArH), 5.13 (s, OCH₂), 4.22-4.34 (m, NHCH₂), 3.66-3.72 (m, NH₃CHCH₂), 1.70-1.80 (m, CH₂CH₃), 0.87 (t, J = 7.5 Hz, CH₂CH₃).

### (R)/(S)-1-((4-((3-(fluorobenzyl)oxy)benzyl)amino)-1-oxobutane-2-aminium chloride (Compound 18)

Using the above Scheme 9, Compound 8 (0.14 g, 0.36 mmol) and 4.0 M HCl in dioxane (0.63 mL, 18.0 mmol) were reacted to synthesize Compound 18 in the form of white powder (0.10 g, 88%): R_{f} = 0.00 (EtOAc 9 : Acetone 1); ¹H NMR (DMSO-d₆, 400 MHz) 8.85 (t, J = 5.3 Hz, C(O)NH), 8.06 (broad, NH₃⁺Cl⁻), 7.38-7.57 (m, ArH), 7.20-7.29 (m, ArH), 6.97-7.02 (m, ArH), 5.13 (s, OCH₂), 4.22-4.34 (m, NHCH₂), 3.66-3.72 (m, NH₃CHCH₂), 1.70-1.80 (m, CH₂CH₃), 0.87 (t, J = 7.5 Hz, CH₂CH₃).

### (R)/(S)-1-((4-((4-(fluorobenzyl)oxy)benzyl)amino)-1-oxobutane-2-aminium chloride (Compound 19)

Using the above Scheme 9, Compound 9 (0.14 g, 0.36 mmol) and 4.0 M HCl in dioxane (0.63 mL, 18.0 mmol) were reacted to synthesize Compound 19 in the form of white powder (0.10 g, 88%): R_{f} = 0.00 (EtOAc 9 : Acetone 1); ¹H NMR (DMSO-d₆, 400 MHz) 8.86 (t, J = 5.7 Hz, C(O)NH), 8.07 (broad, NH₃⁺Cl⁻), 7.63-7.82 (m, ArH), 7.20-7.24 (m, ArH), 6.97-7.03 (m, ArH), 5.13 (s, OCH₂), 4.21-4.34 (m, NHCH₂), 3.66-3.72 (m, NH₃CHCH₂), 1.70-1.82 (m, CH₂CH₃), 0.86 (t, J = 7.5 Hz, CH₂CH₃).

### (R)/(S)-1-oxo-1-((4-((2-(trifluoromethyl)benzyl)oxy)benzyl)amino)butane-2-aminium chloride (Compound 20)

Using the above Scheme 9, Compound 10 (0.10 g, 0.21 mmol) and 4.0 M HCl in dioxane (0.37 mL, 10.7 mmol) were reacted to synthesize Compound 20 in the form of white powder (0.09 g, 99%): R_{f} = 0.00 (EtOAc 9 : Acetone 1); ¹H NMR (DMSO-d₆, 400 MHz) 8.88 (t, J = 5.6 Hz, C(O)NH), 8.15 (broad, NH₃⁺Cl⁻), 7.69-7.83 (m, ArH), 7.56-7.62 (m, ArH), 7.22-7.27 (m, ArH), 6.96-7.02 (m, ArH), 5.22 (s, OCH₂), 4.22-4.34 (m, NHCH₂), 3.68-3.74 (m, NH₃CHCH₂), 1.72-1.81 (m, CH₂CH₃), 0.87 (t, J = 7.4 Hz, CH₂CH₃).

### (R)/(S)-1-oxo-1-((4-((3-(trifluoromethyl)benzyl)oxy)benzyl)amino)butane-2-aminium chloride (Compound 21)

Using the above Scheme 9, Compound 11 (0.09 g, 0.19 mmol) and 4.0 M HCl in dioxane (0.33 mL, 9.7 mmol) were reacted to synthesize Compound 21 in the form of white powder (0.07 g, 93%): R_{f} = 0.00 (EtOAc 9 : Acetone 1); ¹H NMR (DMSO-d₆, 400 MHz) 8.86 (t, J = 5.7 Hz, C(O)NH), 8.09 (broad, NH₃⁺Cl⁻), 7.61-7.82 (m, ArH), 7.20-7.25 (m, ArH), 6.98-7.03 (m, ArH), 5.21 (s, OCH₂), 4.22-4.34 (m, NHCH₂), 3.67-3.72 (m, NH₃CHCH₂), 1.70-1.80 (m, CH₂CH₃), 0.86 (t, J = 7.5 Hz, CH₂CH₃).

### (R)/(S)-1-oxo-1-((4-((4-(trifluoromethyl)benzyl)oxy)benzyl)amino)butane-2-aminium chloride (Compound 22)

Using the above Scheme 9, Compound 12 (0.40 g, 0.86 mmol) and 4.0 M HCl in dioxane (1.49 mL, 42.8 mmol) were reacted to synthesize Compound 22 in the form of white powder (0.33 g, 97%); R_{f} = 0.00 (EtOAc 9 : Acetone 1); ¹H NMR (DMSO-d₆, 400 MHz) 8.89 (t, J = 5.7 Hz, C(O)NH), 8.09 (broad, NH₃⁺Cl⁻), 7.61-7.82 (m, ArH), 7.20-7.25 (m, ArH), 6.98-7.03 (m, ArH), 5.21 (s, OCH₂), 4.22-4.34 (m, NHCH₂), 3.67-3.72 (m, NH₃CHCH₂), 1.70-1.80 (m, CH₂CH₃), 0.86 (t, J = 7.5 Hz, CH₂CH₃).

### (R)/(S)-1-((4-((3-(chlorobenzyl)oxy)benzyl)amino)-1-oxobutane-2-aminium chloride (Compound 23)

Using the above Scheme 9, Compound 13 (0.30 g, 0.7 mmol) and 4.0 M HCl in dioxane (1.20 mL, 34.7 mmol) were reacted to synthesize Compound 23 in the form of white powder (0.25 g, 97%): R_{f} = 0.00 (EtOAc 9 : Acetone 1); ¹H NMR (DMSO-d₆, 400 MHz) 8.91 (t, J = 5.7 Hz, C(O)NH), 8.20 (broad, NH₃⁺Cl⁻), 7.48-7.51 (m, ArH), 7.36-7.45 (m, ArH), 7.19-7.24 (m, ArH), 6.95-7.00 (m, ArH), 5.12 (s, OCH₂), 4.21-4.33 (m, NHCH₂), 3.69-3.74 (m, NH₃CHCH₂), 1.71-1.81 (m, CH₂CH₃), 0.86 (t, J = 7.5 Hz, CH₂CH₃).

### (R)/(S)-1-((4-((4-(chlorobenzyl)oxy)benzyl)amino)-1-oxobutane-2-aminium chloride (Compound 24)

Using the above Scheme 9, Compound 14 (0.10 g, 0.2 mmol) and 4.0 M HCl in dioxane (0.40 mL, 11.5 mmol) were reacted to synthesize Compound 24 in the form of white powder (0.08 g, 98%): R_{f} = 0.00 (EtOAc 9 : Acetone 1); ¹H NMR (DMSO-d₆, 400 MHz) 8.86 (t, J = 5.8 Hz, C(O)NH), 8.15 (broad, NH₃⁺Cl⁻), 7.44-7.46 (m, ArH), 7.19-7.24 (m, ArH), 6.95-7.00 (m, ArH), 5.10 (s, OCH₂), 4.21-4.33 (m, NHCH₂), 3.67-3.73 (m, NH₃CHCH₂), 1.70-1.80 (m, CH₂CH₃), 0.86 (t, J = 7.5 Hz, CH₂CH₃).

### (R)/(S)-1-((4-((3-fluorobenzyl)oxy)benzyl)amino)-1-oxopentane-2-aminium chloride (Compound 25)

Using the above Scheme 9, Compound 15 (0.55 g, 1.3 mmol) and 4.0 M HCl in dioxane (2.22 mL, 64.0 mmol) were reacted to synthesize Compound 25 in the form of white powder (0.43 g, 92%): R_{f} = 0.00 (EtOAc 9 : Acetone 1); ¹H NMR (DMSO-d₆, 400 MHz) 8.90 (t, J = 5.7 Hz, C(O)NH), 8.16 (broad, NH₃⁺Cl⁻), 7.40-7.47 (m, ArH), 7.11-7.30 (m, ArH), 6.96-7.01 (m, ArH), 5.13 (s, OCH₂), 4.24-4.30 (m, NHCH₂), 3.71-3.77 (m, NH₃CHCH₂), 1.65-1.73 (m, CH₂CH₂CH₃), 1.23-1.35 (m, CH₂CH₂CH₃), 0.86 (t, J = 7.3 Hz, CH₂CH₂CH₃).

### (R)/(S)-1-((4-((4-(trifluorobenzyl)oxy)benzyl)amino)-1-oxopentane-2-aminium chloride (Compound 26)

Using the above Scheme 8, Compound 16 (0.65 g, 1.4 mmol) and 4.0 M HCl in dioxane (2.34 mL, 67.5 mmol) were reacted to synthesize Compound 26 in the form of white powder (0.39 g, 70%): R_{f} = 0.00 (EtOAc 9 : Acetone 1); ¹H NMR (DMSO-d₆, 400 MHz) 8.89 (t, J = 5.7 Hz, C(O)NH), 8.15 (broad, NH₃⁺Cl⁻), 7.63-7.79 (m, ArH), 7.20-7.25 (m, ArH), 6.96-7.02 (m, ArH), 5.23 (s, OCH₂), 4.25-4.29 (m, NHCH₂), 3.71-3.77 (m, NH₃CHCH₂), 1.65-1.72 (m, CH₂CH₂CH₃), 1.23-1.35 (m, CH₂CH₂CH₃), 0.86 (t, J = 7.3 Hz, CH₂CH₂CH₃).

### (R)/(S)-2-(methylamino)-N-(4-((4-trifluoromethyl)benzyl)oxy)benzyl)butanamide (Compound 27)

Using the above Scheme 10, Compound 12 (0.40 g, 0.86 mmol), NaH (10 eq.), and CH₃I (10 eq.) were reacted to obtain a methylated compound, and then 4.0 M HCl in dioxane (1.49 mL, 42.8 mmol) was used to remove the Boc group, thereby synthesizing Compound 27 (0.30 g); ¹H NMR (DMSO-d₆, 400 MHz) 8.89 (t, J = 5.7 Hz, C(O)NH), 8.09 (broad, NH₃⁺Cl⁻), 7.61-7.82 (m, ArH), 7.20-7.25 (m, ArH), 6.98-7.03 (m, ArH), 5.18 (s, OCH₂), 4.22-4.34 (m, NHCH₂), 3.67-3.72 (m, NHCHCH₂), 2.39 (s, NHCH₃), 1.70-1.80 (m, CH₂CH₃), 0.86 (t, J = 7.5 Hz, CH₂CH₃).

### (R)/(S)-N-(4-((3-chlorobenzyl)oxy)benzyl)-2-(methylamino)butanamide (Compound 28)

Using the above Scheme 10, Compound 13 (1 eq.), NaH (10 eq.), and CH₃I (10 eq.) were reacted to obtain a methylated compound, and then 4.0 M HCl in dioxane (6 eq.) was used to remove the Boc group, thereby synthesizing Compound 28; ¹H NMR (DMSO-d₆, 400 MHz) 8.92 (t, J = 5.7 Hz, C(O)NH), 8.19 (broad, NH₃⁺Cl⁻), 7.40-7.49 (m, ArH), 7.11-7.32 (m, ArH), 6.96-7.03 (m, ArH), 5.14 (s, OCH₂), 4.24-4.31 (m, NHCH₂), 3.72-3.77 (m, NHCHCH₂), 2.38 (s, NHCH₃), 1.68-1.79 (m, CH₂CH₃), 0.87 (t, J = 7.3 Hz, CH₂CH₃).

### (R)/(S)-N-(4-((4-chlorobenzyl)oxy)benzyl)-2-(methylamino)butanamide (Compound 29)

Using the above Scheme 10, Compound 14 (1 eq.), NaH (10 eq.), and CH₃I (10 eq.) were reacted to obtain a methylated compound, and then 4.0 M HCl in dioxane (6 eq.) was used to remove the Boc group, thereby synthesizing Compound 29; ¹H NMR (DMSO-d₆, 400 MHz) 8.88 (t, J = 5.7 Hz, C(O)NH), 8.07 (broad, NH₃⁺Cl⁻), 7.62-7.81 (m, ArH), 7.21-7.26 (m, ArH), 6.97-7.04 (m, ArH), 5.13 (s, OCH₂), 4.22-4.31 (m, NHCH₂), 3.71-3.77 (m, NHCHCH₂), 2.40 (s, NHCH₃), 1.69-1.79 (m, CH₂CH₃), 0.88 (t, J = 7.3 Hz, CH₂CH₃).

### (R)/(S)-2-(methylamino)-N-(4-(4-trifluoromethyl)benzyl)oxy)benzyl)pentanamide (Compound 30)

Using the above Scheme 10, Compound 12 (0.40 g, 0.86 mmol), NaH (10 eq.), and CH₃I (10 eq.) were reacted to obtain a methylated compound, and then 4.0 M HCl in dioxane (1.49 mL, 42.8 mmol) was used to remove the Boc group, thereby synthesizing Compound 27 (0.30 g); ¹H NMR (DMSO-d₆, 400 MHz) 8.89 (t, J = 5.7 Hz, C(O)NH), 8.09 (broad, NH₃⁺Cl⁻), 7.61-7.82 (m, ArH), 7.20-7.25 (m, ArH), 6.98-7.03 (m, ArH), 5.15 (s, OCH₂), 4.22-4.34 (m, NHCH₂), 3.67-3.72 (m, NHCHCH₂), 2.39 (s, NHCH₃), 1.63-1.72 (m, CH₂CH₂CH₃), 1.22-1.35 (m, CH₂CH₂CH₃), 0.87 (t, J = 7.3 Hz, CH₂CH₂CH₃).

### (R)/(S)-2-(methylamino)-N-(4-((4-trifluoromethyl)benzyl)oxy)benzyl)pentanamide (Compound 31)

Using the above Scheme 11, Compound 26 (0.31 g, 0.7 mmol), triethylamine (0.10 mL, 0.7 mmol), formaldehyde (0.15 mL), and palladium catalyst (0.15 g) were reacted to synthesize Compound 31 in the form of white powder; ¹H NMR (DMSO-d₆, 400 MHz) 8.22 (t, J = 539 Hz, C(O)NH), 7.63-7.79 (m, ArH), 7.17-7.22 (m, ArH), 6.94-6.99 (m, ArH), 5.21 (s, OCH₂), 4.19-4.24 (m, NHCH₂), 2.70-2.76 (NCH), 2.18 (s, N(CH₃)₂), 1.65-1.73 (m, CH₂CH₂CH₃), 1.20-1.35 (m, CH₂CH₂CH₃), 0.86 (t, J = 7.4 Hz, CH₂CH₂CH₃).

### (R)/(S)-tert-butyl-2-((4-(3-fluorobenzyl)oxy)benzyl)carbamoyl)pyrrolidine-1-carboxylate (Compound 34)

Using the above Scheme 11, Compound 33 (2.00 g, 9.3 mmol), 3-fluorobenzyl bromide (2.63 g, 11.2 mmol), and K₂CO₃ (5.14 g, 37.2 mmol) were reacted to synthesize Compound 34 in the form of white powder (3.91 g, 98%): R_{f} = 0.27 (EtOAc 1 : n-hexane 5); ¹H NMR (DMSO-d₆, 400 MHz) 8.27 (t, J = 5.8 Hz, C(O)NH), 7.35-7.50 (m, ArH), 7.20-7.30 (m, ArH), 7.10-7.20 (m, ArH), 6.93 (d, J = 7.8 Hz, Boc-NH), 5.12 (s, OCH₂), 4.00-4.30 (m,NCH, NHCH₂), 3.02-3.45 (NCHCH₂CH₂CH₂), 1.70-2.15 (m,NCHCH₂CH₂CH₂), 1.30 (s, Boc).

### (R)/(S)-tert-butyl-2-((4-((4-fluorobenzyl)oxy)benzyl)carbamoyl)pyrrolidine-1-carboxylate (Compound 35)

Using the above Scheme 11, Compound 33, 4-fluorobenzyl bromide, and K₂CO₃ were reacted to synthesize Compound 35 in the form of white powder; ¹H NMR (DMSO-d₆, 400 MHz) 8.27 (t, J = 5.8 Hz, C(O)NH), 7.35-7.45 (m, ArH), 7.15-7.25 (m, ArH), 7.05-7.10 (m, ArH), 6.85-6.95 (m, ArH), 5.01 (s, OCH₂), 4.20-4.60 (m,NCH, NHCH₂), 2.10-2.50 (NCHCH₂CH₂CH₂), 1.80-2.00 (m,NCHCH₂CH₂CH₂), 1.25 (s, Boc).

### (R)/(S)-tert-butyl-2-((4-((4-(trifluoromethyl)benzyl)oxy)benzyl)carbamoyl)pyrrolidine-1-carboxylate (Compound 36)

Using the above Scheme 11, Compound 33, 4-(trifluoromethyl)benzyl bromide, and K₂CO₃ were reacted to synthesize Compound 36 in the form of white powder; ¹H NMR (DMSO-d₆, 400 MHz) 8.25 (t, J = 5.7 Hz, C(O)NH), 7.70-7.60 (m, ArH), 7.60-7.50 (m, ArH), 7.15-7.25 (m, ArH), 6.85-6.95 (m, ArH), 5.11 (s, OCH₂), 4.20-4.60 (m,NCH, NHCH₂), 2.10-2.40 (NCHCH₂CH₂CH₂), 1.80-1.95 (m,NCHCH₂CH₂CH₂), 1.25 (s, Boc).

### (R)/(S)-tert-butyl-2-((4-(4-(chlorobenzyl)oxy)benzyl)carbamoyl)pyrrolidine-1-carboxylate (Compound 37)

Using the above Scheme 11, Compound 33, 4-chlorobenzyl bromide, and K₂CO₃ were reacted to synthesize Compound 37 in the form of white powder; ¹H NMR (DMSO-d₆, 400 MHz) 8.28 (t, J = 5.9 Hz, C(O)NH), 7.70-7.60 (m, ArH), 7.60-7.50 (m, ArH), 7.15-7.25 (m, ArH), 6.85-6.95 (m, ArH), 5.11 (s, OCH₂), 4.20-4.60 (m,NCH, NHCH₂), 2.10-2.40 (NCHCH₂CH₂CH₂), 1.80-1.95 (m,NCHCH₂CH₂CH₂), 1.27 (s, Boc).

### (R)/(S)-N-(4-((3-fluorobenzyl)oxy)benzyl)pyrrolidine-2-carboxamide hydrochloride (Compound 38)

Using the above Scheme 9, Compound 34 (1.00 g, 2.3 mmol) and 4.0 M HCl in dioxane (7.32 mL, 233.0 mmol) were reacted to synthesize Compound 38 in the form of white powder (0.77 g, 100%): R_{f} = 0.00 (EtOAc 9 : Acetone 1); ¹H NMR (DMSO-d₆, 400 MHz) 8.99 (t, J = 5.5 Hz, C(O)NH), 8.55 (broad, NH₂⁺Cl⁻), 7.39-7.47 (m, ArH), 7.23-7.30 (m, ArH), 7.18-7.23 (m, ArH), 7.10-7.18 (m, ArH), 6.92-7.01 (m, ArH), 5.13 (s, OCH₂), 4.23-4.30 (m, NHCH₂), 4.11-4.19 (m, NHCH), 3.14-3.27 (m, NHCHCH₂CH₂CH₂), 2.24-2.34 (m, NHCHCH₂CHH'CH₂), 1.77-1.93 (m, NHCHCH₂CHH'CH₂).

### (R)/(S)-N-(4-((4-fluorobenzyl)oxy)benzyl)pyrrolidine-2-carboxamide hydrochloride (Compound 39)

Using the above Scheme 9, Compound 35 (1.00 g, 2.3 mmol) and 4.0 M HCl in dioxane (4.05 mL, 117.0 mmol) were reacted to synthesize Compound 39 in the form of white powder (0.84 g, 99%): R_{f} = 0.00 (EtOAc 9 : Acetone 1); ¹H NMR (DMSO-d₆, 400 MHz) 9.04 (t, J = 5.7 Hz, C(O)NH), 8.55 (broad, NH₂⁺Cl⁻), 7.46-7.53 (m, ArH), 7.18-7.26 (m, ArH), 6.95-7.01 (m, ArH), 5.08 (s, OCH₂), 4.25-4.31 (m, NHCH₂), 4.12-4.21 (m, NHCH), 3.13-3.29 (m, NHCHCH₂CHHCH₂), 2.25-2.35 (m, NHCHCH₂CHHCH₂), 1.77-1.94 (m, NHCHCH₂CHHCH₂).

### (R)/(S)-N-(4-((4-(trifluoromethyl)benzyl)oxy)benzyl)pyrrolidine-2-carboxamide hydrochloride (Compound 40)

Using the above Scheme 9, Compound 36 (1.00 g, 2.09 mmol) and 4.0 M HCl in dioxane (3.63 mL, 104.0 mmol) were reacted to synthesize Compound 40 in the form of white powder (0.85 g, 98%): R_{f} = 0.00 (EtOAc 9 : Acetone 1); ¹H NMR (DMSO-d₆, 400 MHz) 8.98 (t, J = 5.8 Hz, C(O)NH), 8.56 (broad, NH₂⁺Cl⁻), 7.63-7.79 (m, ArH), 7.19-7.25 (m, ArH), 6.97-7.03 (m, ArH), 5.23 (s, OCH₂), 4.26-4.30 (m, NHCH₂), 4.12-4.18 (m, NHCH), 3.13-3.27 (m, NHCHCH₂CH₂CH₂), 2.23-2.35 (m, NHCHCH₂CHH'CH₂), 1.77-1.93 (m, NHCHCH₂CHH'CH₂).

### (R)/(S)-N-(4-((4-chlorobenzyl)oxy)benzyl)pyrrolidine-2-carboxamide hydrochloride (Compound 41)

Using the above Scheme 9, Compound 37 (1.00 g, 2.09 mmol) and 4.0 M HCl in dioxane (3.63 mL, 104.0 mmol) were reacted to synthesize Compound 41 in the form of white powder (0.85 g, 98%): R_{f} = 0.00 (EtOAc 9 : Acetone 1); ¹H NMR (DMSO-d₆, 400 MHz) 8.97 (t, J = 5.4 Hz, C(O)NH), 8.57 (broad, NH₂⁺Cl⁻), 7.40-7.48 (m, ArH), 7.20-7.29 (m, ArH), 7.18-7.23 (m, ArH), 7.08-7.18 (m, ArH), 6.95-7.05 (m, ArH), 5.11 (s, OCH₂), 4.20-4.31 (m, NHCH₂), 4.09-4.19 (m, NHCH), 3.14-3.27 (m, NHCHCH₂CH₂CH₂), 2.25-2.36 (m, NHCHCH₂CHH'CH₂), 1.70-1.83 (m, NHCHCH₂CHH'CH₂).

### (R)/(S)-1-methyl-N-(4-((3-fluorobenzyl)oxy)benzyl)pyrrolidine-2-carboxamide (Compound 42)

Using the above Scheme 11, Compound 38 (0.31 g, 0.7 mmol), formaldehyde (0.15 mL), and palladium catalyst (0.15 g) were reacted to synthesize Compound 42 in the form of white powder; ¹H NMR (DMSO-d₆, 400 MHz) 7.57 (t, J = 5.2 Hz, C(O)NH), 7.36-7.43 (m, ArH), 7.17-7.28 (m, ArH), 7.01-7.08 (m, ArH), 6.95-7.00 (m, ArH), 5.05 (s, OCH₂), 4.33-4.37 (m, NHCH₂), 3.10-3.17 (m, NCHCHHCH₂CH₂), 2.85-2.91 (m, NCHCHHCH₂CH₂), 2.34 (s, NCH₃), 2.17-2.32 (m, NCHCH₂CH₂CH₂), 1.82-1.92 (m, NCHCH₂CH₂CH₂), 1.67-1.82 (m, NCHCH₂CH₂CH₂).

### (R)/(S)-1-methyl-N-(4-((4-(trifluoromethyl)benzyl)oxy)benzyl)pyrrolidine-2-carboxamide (Compound 43)

Using the above Scheme 11, Compound 40 (0.31 g, 0.7 mmol), triethylamine (0.10 mL, 0.7 mmol), formaldehyde (0.15 mL), and palladium catalyst (0.15 g) were reacted to synthesize Compound 43 in the form of white powder; ¹H NMR (DMSO-d₆, 400 MHz) 7.62-7.67 (m, ArH), 7.52-7.62 (t, J = 5.4 Hz, C(O)NH, m, ArH), 7.18-7.23 (m, ArH), 6.90-6.96 (m, ArH), 5.12 (s, OCH₂), 4.33-4.45 (m, NHCH₂), 3.00-3.10 (m, NCHCH₂CH₂CH₂), 2.90-2.98 (m, NCHCHHCH₂CH₂), 2.37 (s, NCH₃), 2.30-2.40 (m, NCHCH₂CH₂CH₂), 2.18-2.30 (m, NCHCHHCH₂CH₂).

### (R)/(S)-1-methyl-N-(4-((4-chlorobenzyl)oxy)benzyl)pyrrolidine-2-carboxamide (Compound 44)

Using the above Scheme 11, Compound 41 (0.31 g, 0.7 mmol), formaldehyde (0.15 mL), and palladium catalyst (0.15 g) were reacted to synthesize Compound 44 in the form of white powder; ¹H NMR (DMSO-d₆, 400 MHz) 7.75 (t, J = 5.4 Hz, C(O)NH), 7.60-7.67 (m, ArH), 7.52-7.62 (m, ArH), 7.18-7.23 (m, ArH), 6.90-7.00 (m, ArH), 5.11 (s, OCH₂), 4.34-4.45 (m, NHCH₂), 3.00-3.09 (m, NCHCH₂CH₂CH₂), 2.90-2.99 (m, NCHCHHCH₂CH₂), 2.35 (s, NCH₃), 2.28-2.40 (m, NCHCH₂CH₂CH₂), 2.18-2.30 (m, NCHCHHCH₂CH₂).

### (R)/(S)-tert-butyl-3-((4((3-fluorobenzyl)oxy)benzyl)carbamoyl)pyrolidine-1-carboxylate (Compound 46)

Using the above Scheme 7, Compound 45 (1.00 g, 4.6 mmol), NMM (0.77 mL, 7.0 mmol), IBCF (0.76 mL, 5.8 mmol), and 4-[(3-fluorophenyl)methoxy]-benzenemethaneamine hydrochloride (1.47 g, 5.5 mmol) were reacted to synthesize Compound 46 in the form of white powder (1.97 g, 99%): R_{f} = 0.30 (EtOAc 1 : n-hexane 1); ¹H NMR (DMSO-d₆, 400 MHz) 7.57 (t, J = 5.2 Hz, C(O)NH), 7.32-7.39 (m, ArH), 7.11-7.24 (m, ArH), 6.89-6.94 (m, ArH), 5.07 (s, OCH₂), 4.33-4.37 (m, NHCH₂), 3.21-3.68 (m, NCH₂CHCH₂CH₂), 2.85-2.98 (m, NCH₂CHCH₂CH₂), 2.00-2.12 (m, NCH₂CHCH₂CH₂), 1.45 (s, Boc).

### (R)/(S)-tert-butyl-3-((4((4-(trifluoromethyl)benzyl)oxy)benzyl)carbamoyl)pyrrolidine-1-carboxylate (Compound 47)

Using the above Scheme 7, Compound 45 (1.00 g, 4.6 mmol), NMM (0.77 mL, 7.0 mmol), IBCF (0.76 mL, 5.8 mmol), and 4-[(4-(trifluoromethyl)phenyl)methoxy]-benzenemethaneamine hydrochloride (1.75 g, 5.5 mmol) were reacted to synthesize Compound 47 in the form of white powder (2.20 g, 99%): R_{f} = 0.33 (EtOAc 1 : n-hexane 1); ¹H NMR (DMSO-d₆, 400 MHz) 7.78 (t, J = 5.3 Hz, C(O)NH), 7.63-7.67 (m, ArH), 7.52-7.57 (m, ArH), 7.18-.23 (m, ArH), 6.90-6.95 (m, ArH), 5.12 (s, OCH₂), 4.37-4.42 (m, NHCH₂), 3.53-3.67 (m, NCH₂CHCH₂CH₂), 3.47-3.53 (m, NCH₂CHCH₂CH₂), 2.78-2.89 (m, NCH₂CHCH₂CH₂), 2.06-2.13 (m, NCH₂CHCH₂CH₂), 1.45 (s, Boc).

### (R)/(S)-tert-butyl-3-((4-(4-chlorobenzyl)oxy)benzyl)carbamoyl)pyrrolidine-1-carboxylate (Compound 48)

Using the above Scheme 7, Compound 45 (1.00 g, 4.6 mmol), NMM (0.77 mL, 7.0 mmol), IBCF (0.76 mL, 5.8 mmol), and 4-[(4-(trifluoromethyl)phenyl)methoxy]-benzenemethaneamine hydrochloride (1.75 g, 5.5 mmol) were reacted to synthesize Compound 48 in the form of white powder (2.20 g, 99%): R_{f} = 0.33 (EtOAc 1 : n-hexane 1); ¹H NMR (DMSO-d₆, 400 MHz) 7.80 (t, J = 5.4 Hz, C(O)NH), 7.63-7.67 (m, ArH), 7.52-7.57 (m, ArH), 7.18-7.23 (m, ArH), 6.90-6.95 (m, ArH), 5.12 (s, OCH₂), 4.37-4.42 (m, NHCH₂), 3.53-3.67 (m, NCH₂CHCH₂CH₂), 3.47-3.53 (m, NCH₂CHCH₂CH₂), 2.78-2.89 (m, NCH₂CHCH₂CH₂), 2.06-2.13 (m, NCH₂CHCH₂CH₂), 1.45 (s, Boc).

### (R)/(S)-2-((4-((3-fluorobenzyl)oxy)benzyl)carbamoyl)pyrrolidi-1-nium chloride (Compound 49)

Using the above Scheme 9, Compound 46 (1.00 g, 2.3 mmol) and 4.0 M HCl in dioxane (4.05 mL, 116.7 mmol) were reacted to synthesize Compound 49 in the form of white powder (0.84 g, 99%): R_{f} = 0.00 (EtOAc 9 : Acetone 1); ¹H NMR (DMSO-d₆, 400 MHz) 9.13 (broad, NH₂⁺Cl⁻), 8.65 (t, J = 5.6 Hz, C(O)NH), 7.40-7.48 (m, ArH), 7.23-7.30 (m, ArH), 7.12-7.21 (m, ArH), 6.94-7.00 (m, ArH), 5.12 (s, OCH₂), 4.15-4.27 (m, NHCH₂), 3.04-3.32 (m, NHCH₂CHCH₂CH₂), 1.88-2.20 (m, NHCH₂CHCH₂CH₂).

### (R)/(S)-2-((4-((4-(trifluoromethyl)benzyl)oxy)benzyl)carbamoyl)pyrrolidi-1-nium chloride (Compound 50)

Using the above Scheme 9, Compound 47 (1.00 g, 2.09 mmol) and 4.0 M HCl in dioxane (3.63 mL, 104.5 mmol) were reacted to synthesize Compound 50 in the form of white powder (0.86 g, 99%): R_{f} = 0.00 (EtOAc 9 : Acetone 1); ¹H NMR (DMSO-d₆, 400 MHz) 9.13 (broad, NH₂⁺Cl⁻), 8.65 (t, J = 5.6 Hz, C(O)NH), 7.60-7.71 (m, ArH), 7.20-7.27 (m, ArH), 6.95-7.01 (m, ArH), 5.20 (s, OCH₂), 4.28-4.35 (m, NHCH₂), 3.36-3.56 (m, NHCH₂CHCH₂CH₂), 2.27-2.39 (m, NHCH₂CHCH₂CH₂), 2.10-2.20 (m, NHCH₂CHCH₂CH₂).

### (R)/(S)-2-((4-(4-chlorobenzyl)oxy)benzyl)carbamoyl)pyrrolidi-1-nium chloride (Compound 51)

Using the above Scheme 9, Compound 48 (1.00 g, 2.09 mmol) and 4.0 M HCl in dioxane (3.63 mL, 104.5 mmol) were reacted to synthesize Compound 51 in the form of white powder (0.86 g, 99%): R_{f} = 0.00 (EtOAc 9 : Acetone 1); ¹H NMR (DMSO-d₆, 400 MHz) 9.12 (broad, NH₂⁺Cl⁻), 8.63 (t, J = 5.6 Hz, C(O)NH), 7.60-7.70 (m, ArH), 7.20-7.29 (m, ArH), 6.92-7.01 (m, ArH), 5.18 (s, OCH₂), 4.27-4.35 (m, NHCH₂), 3.38-3.56 (m, NHCH₂CHCH₂CH₂), 2.25-2.40 (m, NHCH₂CHCH₂CH₂), 2.10-2.20 (m, NHCH₂CHCH₂CH₂).

### (R)/(S)-1-methyl-N-(4-((3-fluorobenzyl)oxy)benzyl)pyrrolidine-3-carboxamide (Compound 52)

Using the above Scheme 11, Compound 49 (0.14 g, 0.4 mmol), formaldehyde (0.03 mL), and palladium catalyst (0.07 g) were reacted to synthesize Compound 52 in the form of white powder; ¹H NMR (DMSO-d₆, 400 MHz) 7.57 (t, J = 5.2 Hz, C(O)NH), 7.36-7.43 (m, ArH), 7.16-7.28 (m, ArH), 7.01-7.08 (m, ArH), 6.94-6.99 (m, ArH), 5.11 (s, OCH₂), 4.29-4.31 (m, NHCH₂), 2.93-3.05 (m, NHCH₂CHCH₂CH₂), 2.78-2.85 (m, NHCH₂CHCH₂CH₂), 2.50-2.64 (m, NHCH₂CHCH₂CH₂), 2.39 (s, NCH₃), 2.02-2.15 (m, m, NHCH₂CHCH₂CH₂).

### (R)/(S)-1-methyl-N-(4-((4-(trifluoromethyl)benzyl)oxy)benzyl)pyrrolidine-3-carboxamide (Compound 53)

Using the above Scheme 11, Compound 50 (0.14 g, 0.4 mmol), formaldehyde (0.03 mL), and palladium catalyst (0.07 g) were reacted to synthesize Compound 53 in the form of white powder; ¹H NMR (DMSO-d₆, 400 MHz) 7.62-7.69 (m, ArH), 7.52-7.57 (m, ArH), 7.17-7.23 (m, ArH), 7.02 (t, J = 5.4 Hz, C(O)NH), 6.89-6.95 (m, ArH), 5.12 (s, OCH₂), 4.30-4.45 (m, NHCH₂), 2.75-2.95 (m, NCH₂CH₂CHCH₂), 2.27-2.50 (m, NCH₂CH₂CHCH₂), 2.35 (s, NCH₃), 1.95-2.25 (m, NCH₂CHCH₂CH₂).

### (R)/(S)-1-methyl-N-(4-((4-chlorobenzyl)oxy)benzyl)pyrrolidine-3-carboxamide (Compound 54)

Using the above Scheme 11, Compound 51 (0.14 g, 0.4 mmol), formaldehyde (0.03 mL), and palladium catalyst (0.07 g) were reacted to synthesize Compound 54 in the form of white powder; ¹H NMR (DMSO-d₆, 400 MHz) 7.60-7.69 (m, ArH), 7.50-7.57 (m, ArH), 7.17-7.25 (m, ArH), 7.05 (t, J = 5.4 Hz, C(O)NH), 6.89-6.95 (m, ArH), 5.10 (s, OCH₂), 4.32-4.43 (m, NHCH₂), 2.75-2.95 (m, NCH₂CH₂CHCH₂), 2.25-2.51 (m, NCH₂CH₂CHCH₂), 2.34 (s, NCH₃), 1.94-2.27 (m, NCH₂CHCH₂CH₂).

### (R)/(S)-tert-butyl(1-((4-((3,4-chlorobenzyl)oxy)benzyl)amino)-1-oxobuta-2-nyl)carbamate (Compound 55)

Using the above Scheme 8, Compound 5 (0.20 g, 0.7 mmol), 3,4-chlorobenzyl bromide (0.09 mL, 0.7 mmol), and K₂CO₃ (0.36 g, 2.6 mmol) were reacted to synthesize Compound 55 in the form of white powder (0.28 g, 100%): R_{f} = 0.50 (EtOAc 1 : n-hexane 1); ¹H NMR (DMSO-d₆, 400 MHz) 8.21 (t, J = 5.9 Hz, C(O)NH), 7.42-7.52 (m, ArH), 7.13-7.20 (m, ArH), 6.90-6.95 (m, ArH), 6.80 (d, J = 8.0 Hz, Boc-NH), 5.08 (s, OCH₂), 4.13-4.27 (m, NHCH₂), 3.79-3.87 (m, NHCHCH₂), 1.45-1.68 (m, CH₂CH₃), 1.37 (s, Boc), 0.82 (t, J = 7.3 Hz, CH₂CH₃).

### (R)/(S)-tert-butyl(1-oxo-1-((4-((4-(trifluoromethyl)benzyl)oxy)benzyl)amino)penta-2-nyl)carbamate (Compound 56)

Using the above Scheme 8, Compound 6 (1.00 g, 3.2 mmol), 3,4-dichlorobenzyl bromide (0.57 mL, 3.9 mmol), and K₂CO₃ (1.79 g, 13.0 mmol) were reacted to synthesize Compound 56 in the form of white powder (0.75 g, 50%): R_{f} = 0.24 (EtOAc 1 : n-hexane 2); ¹H NMR (DMSO-d₆, 400 MHz) 8.22 (t, J = 5.9 Hz, C(O)NH), 7.60-7.75 (m, ArH), 7.38-7.48 (m, ArH), 7.10-7.25 (m, ArH), 6.85-6.98 (m, ArH), 6.82 (d, J = 7.8 Hz, Boc-NH), 5.12 (s, OCH₂), 4.10-4.30 (m, NHCH₂), 3.78-3.90 (m, NHCHCH₂), 1.41-1.70 (m, CH₂CH₃), 1.38 (s, Boc), 0.87 (t, J = 7.8 Hz, CH₂CH₃).

### (R)/(S)-1-((4-(3,4-dichlorobenzyl)oxy)benzyl)amino)-1-oxobutane-2-aminium chloride (Compound 57)

Using the above Scheme 9, Compound 55 (0.29 g, 0.6 mmol) and 4.0 M HCl in dioxane (1.05 mL, 30.1 mmol) were reacted to synthesize Compound 57 in the form of white powder (0.24 g, 95%): R_{f} = 0.30 (CH₂Cl₂ 9 : MeOH 1); ¹H NMR (DMSO-d₆, 400 MHz) 8.85 (t, J = 5.6 Hz, C(O)NH), 8.10 (broad, NH₃⁺Cl⁻), 7.63-7.75 (m, ArH), 7.40-7.50 (m, ArH), 7.18-7.30 (m, ArH), 6.93-7.03 (m, ArH), 5.13 (s, OCH₂), 4.20-4.38 (m, NHCH₂), 3.65-3.78 (m, NH₃CHCH₂), 1.70-1.80 (m, CH₂CH₃), 0.87 (t, J = 7.4 Hz, CH₂CH₃).

### (R)/(S)-1-((4-((3,4-dichlorobenzyl)oxy)benzyl)amino)-1-oxopentane-2-aminium chloride (Compound 58)

Using the above Scheme 9, Compound 56 (0.65 g, 1.4 mmol) and 4.0 M HCl in dioxane (2.34 mL, 67.5 mmol) were reacted to synthesize Compound 58 in the form of white powder (0.39 g, 70%): R_{f} = 0.00 (EtOAc 9 : Acetone 1); ¹H NMR (DMSO-d₆, 400 MHz) 8.99 (t, J = 7.3 Hz, C(O)NH), 8.26 (broad, NH₃⁺Cl⁻), 7.60-7.78 (m, ArH), 7.38-7.48 (m, ArH), 7.10-7.40 (m, ArH), 6.90-7.10 (m, ArH), 5.12 (s, OCH₂), 4.18-4.38 (m, NHCH₂), 3.70-3.83 (m, NH₃CHCH₂), 1.60-1.80 (m, CH₂CH₂CH₃), 1.20-1.40 (m, CH₂CH₂CH₃), 0.86 (t, J = 9.6 Hz, CH₂CH₂CH₃).

### (R)/(S)-2-(dimethylamino)-N-(4-((4-trifluoromethyl)benzyl)oxy)benzyl)butanamide (Compound 59)

Using the above Scheme 11, Compound 22 (0.10 g, 0.2 mmol), triethylamine (0.20 mL, 1.4 mmol), formaldehyde (0.03 mL), and palladium catalyst (0.10 g) were reacted to synthesize Compound 59 in the form of white powder; ¹H NMR (DMSO-d₆, 400 MHz) 8.23 (t, J = 5.9 Hz, C(O)NH), 7.73-7.79 (m, ArH), 7.62-7.70 (m, ArH), 7.16-7.23 (m, ArH), 6.93-6.99 (m, ArH), 5.21 (s, OCH₂), 4.17-4.24 (m, NHCH₂), 2.70-2.76 (NCH), 2.18 (s, N(CH₃)₂), 1.48-1.63 (m, CH₂CH₃), 0.78 (t, J = 7.4 Hz, CH₂CH₃).

### (R)/(S)-N-(4-((3,4-dichlorobenzyl)oxy)benzyl)-2-(dimethylamino)butanamide (Compound 60)

Using the above Scheme 11, Compound 57 (0.20 g, 0.5 mmol), triethylamine (0.40 mL, 2.9 mmol), formaldehyde (0.11 mL), and palladium catalyst (0.04 g) were reacted to synthesize Compound 60 in the form of white powder; ¹H NMR (DMSO-d₆, 400 MHz) 8.23 (t, J = 5.8 Hz, C(O)NH), 7.63-7.72 (m, ArH), 7.30-7.47 (m, ArH), 7.15-7.22 (m, ArH), 6.93-6.99 (m, ArH), 5.11 (s, OCH₂), 4.10-4.30 (m, NHCH₂), 2.80-2.90 (NCH), 2.19 (s, N(CH₃)₂), 1.40-1.63 (m, CH₂CH₂CH₃), 1.25-1.30 (m, CH₂CH₂CH₃), 0.86 (t, J = 7.4 Hz, CH₂CH₂CH₃).

### (R)/(S)-N-(4-((3,4-dichlorobenzyl)oxy)benzyl)-2-(dimethylamino)butanamide (Compound 61)

Using the above Scheme 11, Compound 58 (0.31 g, 0.7 mmol), triethylamine (0.10 mL, 0.7 mmol), formaldehyde (0.15 mL), and palladium catalyst (0.15 g) were reacted to synthesize Compound 61 in the form of white powder; ¹H NMR (DMSO-d₆, 400 MHz) 8.23 (t, J = 5.7 Hz, C(O)NH), 7.60-7.72 (m, ArH), 7.30-7.49 (m, ArH), 7.10-7.25 (m, ArH), 6.92-7.00 (m, ArH), 5.15 (s, OCH₂), 4.10-4.30 (m, NHCH₂), 2.79-2.89 (NCH), 2.18 (s, N(CH₃)₂), 1.40-1.60 (m, CH₂CH₂CH₃), 1.23-1.31 (m, CH₂CH₂CH₃), 0.87 (t, J = 7.4 Hz, CH₂CH₂CH₃).

### (R)/(S)-2-(ethylamino)-N-(4-((4-trifluoromethyl)benzyl)oxy)benzyl)butanamide (Compound 62)

Using the above Scheme 12, Compound 22 (0.10 g, 0.3 mmol), triethylamine (0.21 mL, 1.5 mmol), and iodoethane (0.10 mL) were reacted to synthesize Compound 62 in the form of white powder; ¹H NMR (DMSO-d₆, 400 MHz) 7.61-7.67 (m, ArH), 7.51-7.60 (m, ArH), 7.17-7.25 (m, ArH), 6.89-6.95 (m, ArH), 5.12 (s, OCH₂), 4.34-4.45 (m, NHCH₂), 3.03-3.09 (NCH), 2.52-2.67 (m, CH₂CH₂CH₃), 1.75-1.87 (m, CHHCH₃), 1.55-1.68 (m, CHHCH₃, CH₂CH₂CH₃), 1.06 (t, J = 7.1 Hz, CH₂CH₃), 0.95 (t, J = 7.5 Hz, CH₂CH₂CH₃).

### (R)/(S)-N-(4-((3,4-dichlorobenzyl)oxy)benzyl-2-(ethylamino)butanamide (Compound 63)

Using the above Scheme 12, Compound 57 (0.10 g, 0.7 mmol), triethylamine (0.21 mL, 1.5 mmol), and iodoethane (0.10 mL) were reacted to synthesize Compound 63 in the form of white powder; ¹H NMR (DMSO-d₆, 400 MHz) 7.61-7.70 (m, ArH), 7.50-7.60 (m, ArH), 7.25-7.38 (m, ArH), 6.99-7.15 (m, ArH), 5.10 (s, OCH₂), 4.30-4.45 (m, NHCH₂), 3.10-3.25 (NCH), 2.48-2.67 (m, CH₂CH₂CH₃), 1.75-1.88 (m, CHHCH₃), 1.53-1.69 (m, CHHCH₃, CH₂CH₂CH₃), 1.06 (t, J = 7.1 Hz, CH₂CH₃), 0.93 (t, J = 7.4 Hz, CH₂CH₂CH₃).

### (R)/(S)-2-(ethylamino)-N-(4-((4-(trifluoromethyl)benzyl)oxy)benzyl)pentanamide (Compound 64)

Using the above Scheme 12, Compound 26 (0.13 g, 0.3 mmol), triethylamine (0.26 mL, 1.9 mmol), and iodoethane (0.02 mL) were reacted to synthesize Compound 64 in the form of white powder; ¹H NMR (DMSO-d₆, 400 MHz) 7.63-7.80 (m, ArH), 7.18-7.30 (m, ArH), 6.90-7.00 (m, ArH), 5.20 (s, OCH₂), 4.19-4.38 (m, NHCH₂), 3.35-3.42 (m, CHCH₂CH₂CH₃), 1.63-1.73 (m, NH), 1.33-1.48 (m, CHHCH₂CH₃CH₂CH₃), 1.15-1.19 (m, CHHCH₂CH₃), 1.10-1.15 (m, CH₂CH₃), 0.90 (t, J = 7.1 Hz, CH₂CH₂CH₃).

### (R)/(S)-N-(4-((3,4-dichlorobenzyl)oxy)benzyl-2-(ethylamino)pentanamide (Compound 65)

Using the above Scheme 12, Compound 58 (0.20 g, 0.5 mmol), triethylamine (0.40 mL, 2.9 mmol), and iodotetaine (0.04 mL, 0.4 mmol) were reacted to synthesize Compound 65 in the form of white powder; ¹H NMR (DMSO-d₆, 400 MHz) 8.26 (t, J = 5.9 Hz, C(O)NH), 7.68-7.72 (m, ArH), 7.62-7.68 (m, ArH), 67.40-7.46 (m, ArH), 7.14-7.20 (m, ArH), 6.91-6.99 (m, ArH), 5.11 (s, OCH₂), 4.18-4.25 (m, NHCH₂), 2.92-2.98 (NCH), 2.31-2.48 (m, CH₂CH₂CH₃), 1.35-1.50 (m, CH₂CH₃), 1.20-1.35 (m, CH₂CH₂CH₃), 0.96 (t, J =7.1 Hz, CH₂CH₃), 0.84 (t, J = 7.2 Hz, CH₂CH₂CH₃).

### (R)/(S)-2-(diethylamino)-N-(4-((4-(trifluoromethyl)benzyl)oxy)benzyl)butanamide (Compound 66)

Using the above Scheme 12, Compound 22 (0.10 g, 0.3 mmol), triethylamine (0.21 mL, 1.5 mmol), and iodoethane (0.10 mL) were reacted to synthesize Compound 66 in the form of white powder; ¹H NMR (DMSO-d₆, 400 MHz) 8.20 (t, J = 5.4 Hz, NHCH₂), 7.57-7.70 (m, ArH), 7.10-7.22(m, ArH), 6.88-7.02 (m, ArH), 5.25 (s, OCH₂), 4.08-4.23 (m, NHCH₂), 3.05-3.13 (m, CHCH₂CH₃), 2.52-2.63 (m, CH₂CH₃), 2.35-2.45 (m, CH₂CH₃), 1.42-1.67 (m, CH₂CH₃), 0.93 (t, J = 7.0 Hz, CH₂CH₃, CH₂CH₃), 0.84 (t, J = 7.4 Hz, CH₂CH₃).

### (R)/(S)-N-(4-((3,4-dichlorobenzyl)oxy)benzyl-2-(diethylamino)butanamide (Compound 67)

Using the above Scheme 12, Compound 57 (0.10 g, 0.7 mmol), triethylamine (0.21 mL, 1.5 mmol), and iodoethane (0.10 mL) were reacted to synthesize Compound 67 in the form of white powder; ¹H NMR (DMSO-d₆, 400 MHz) 8.16 (t, J = 5.3 Hz, NHCH₂), 7.57-7.72 (m, ArH), 7.10-7.25(m, ArH), 6.93-7.02 (m, ArH), 5.21 (s, OCH₂), 4.10-4.26 (m, NHCH₂), 3.10-3.18 (m, CHCH₂CH₃), 2.50-2.63 (m, CH₂CH₃), 2.35-2.48 (m, CH₂CH₃), 1.35-1.55 (m, CH₂CH₃), 0.92 (t, J = 7.0 Hz, CH₂CH₃, CH₂CH₃), 0.84 (t, J = 7.4 Hz, CH₂CH₃).

### (R)/(S)-2-(diethylamino)-N-(4-((4-(trifluoromethyl)benzyl)oxy)benzyl)pentanamide Compound (68)

Using the above Scheme 12, Compound 26 (0.13 g, 0.3 mmol), triethylamine (0.26 mL, 1.9 mmol), and iodoethane (0.02 mL) were reacted to synthesize 68 in the form of white powder; ¹H NMR (DMSO-d₆, 400 MHz) 8.16 (t, J = 5.4 Hz, NHCH₂), 7.60-7.80 (m, ArH), 7.15-7.25(m, ArH), 6.90-7.00 (m, ArH), 5.21 (s, OCH₂), 4.12-4.26 (m, NHCH₂), 3.09-3.15 (m, CHCH₂CH₂CH₃), 2.52-2.63 (m, CH₂CH₃), 2.38-2.48 (m, CH₂CH₃), 1.40-1.60 (m, CH₂CH₂CH₃), 1.20-1.31 (m, CH₂CH₂CH₃), 0.93 (t, J = 7.1 Hz, CH₂CH₃, CH₂CH₃), 0.86 (t, J = 7.3 Hz, CH₂CH₃).

### (R)/(S)-N-(4-((3,4-dichlorobenzyl)oxy)benzyl-2-(diethylamino)pentanamide (Compound 69)

Using the above Scheme 12, Compound 58 (0.20 g, 0.5 mmol), triethylamine (0.40 mL, 2.9 mmol), and iodotetaine (0.04 mL, 0.4 mmol) were reacted to synthesize Compound 69 in the form of white powder; ¹H NMR (DMSO-d₆, 400 MHz) 8.20 (t, J = 5.4 Hz, NHCH₂), 7.60-7.80 (m, ArH), 7.13-7.22(m, ArH), 6.90-7.02 (m, ArH), 5.20 (s, OCH₂), 4.12-4.25 (m, NHCH₂), 3.10-3.15 (m, CHCH₂CH₂CH₃), 2.50-2.63 (m, CH₂CH₃), 2.37-2.49 (m, CH₂CH₃), 1.40-1.60 (m, CH₂CH₂CH₃), 1.20-1.33 (m, CH₂CH₂CH₃), 0.92 (t, J = 7.1 Hz, CH₂CH₃, CH₂CH₃), 0.86 (t, J = 7.4 Hz, CH₂CH₃).

### Test Example 1: Antifungal activity assay (MIC analysis)

*In vitro* antimicrobial activity (MIC) against human pathogenic fungi was carried out for the 41 compounds synthesized in the above preparation examples, and the results are shown in Table 1 below. In order to measure the minimum inhibitory concentration (MIC), strains *Candida albicans* SC5314 (ATCC), *Candida glabrata* BG2 (ATCC), and *Cryptococcus neoformans var. grubii* H99 (ATCC) were used. All the strains used in the test examples of the present invention were purchased from Korean Culture Center of Microorganisms (KCCM) and ATCC, and used.

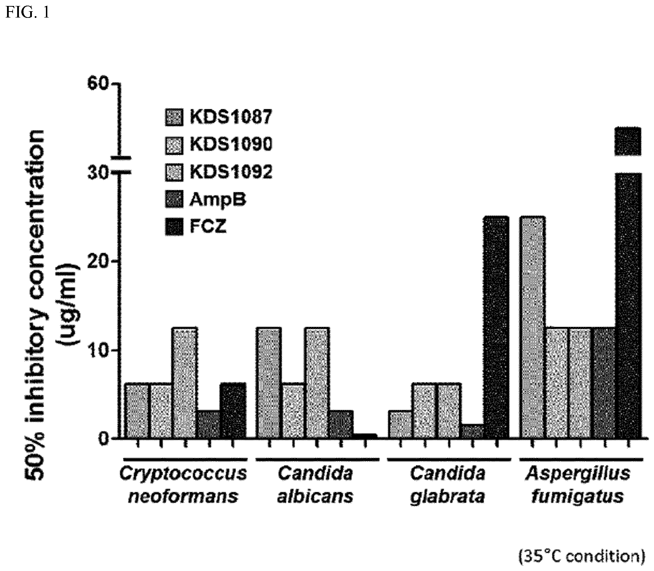

The antifungal activity assay was performed, according to the National Committee for Clinical Laboratory Standards (NCCLS) method, by inoculating an inoculum of 5 × 10² to 2.5 × 10 cells/mL into a 2-fold dilution series of the sample solution to be tested, performing culture in RPMI 1640 medium (Thermo Fisher) in such a way that the Candidas were cultured for 48 hours and the *Cryptococcus neoformans* strain was cultured for 72 hours, and then visually observing viability thereof. As a control group, amphotericin B and fluconazole, which are known to have excellent antifungal inhibitory activity with a problem of human toxicity, were used. Specifically, in order to prepare a medium first, 8.4 g of RPMI-1640 powder (Sigma; R1383-1L) was added to 900 ml of distilled water. Next, 34.5 g of MOPS was added thereto, to bring the final concentration to 0.165 M. Then, 100 ml of 20% glucose was added thereto, and then the pH of the resultant was adjusted to 7 using NaOH. Next, after performing filtration with a filter, the filtrate was stored in a refrigeration condition at 4°C for use in the next experiment. The experimental method and procedure for the MIC assay were as follows: (a) A culture of *Cryptococcus neoformans* or *Candida albicans* cultured overnight was diluted to be OD600 = 1.0, (b) the resultant was diluted in a prepared RPMI-1640 medium to final OD600 = 0.01 (100-fold dilution); (c) in a case of*Aspergillus fumigatus,* a diluent was prepared at a concentration of 1 × 10⁵ conidia/ml; (d) a 96-well plate was used and 100 µl of the solution was added to each well, so that 2 µl of diluted drug was prepared; (e) 200 µl of the solution was added to a well into which the highest concentration of drug was to be added, and dilution was performed by serial dilution; (f) after performing static culture for 48 hours at 37°C, a degree of growth was measured at OD600 and a comparison was made. The results are shown in Table 1 and FIG. 1. As can be identified in the following Table 1 and FIG. 1, the compounds of the present invention exhibited very excellent inhibitory activity against pathogenic fungi used.

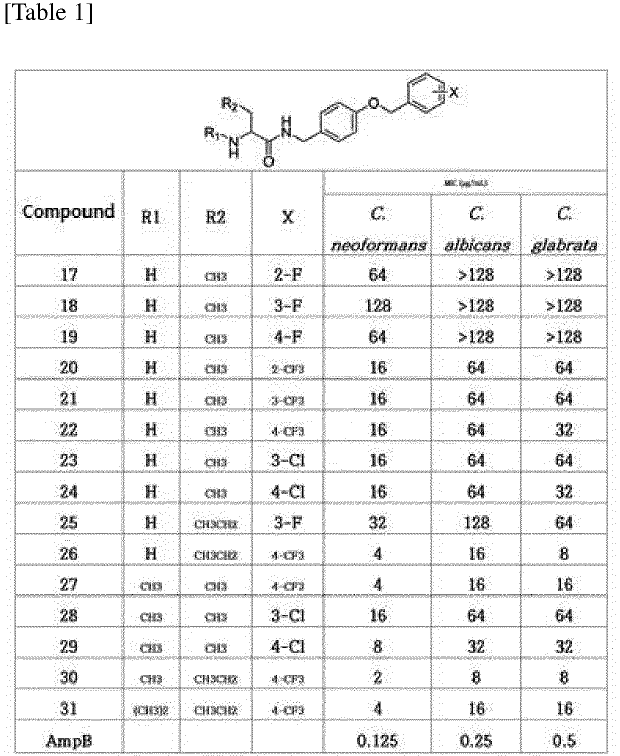

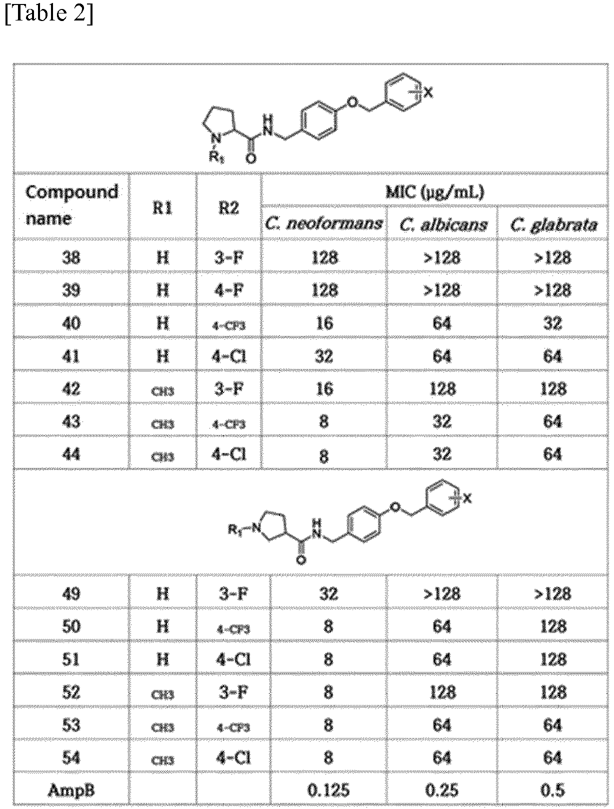

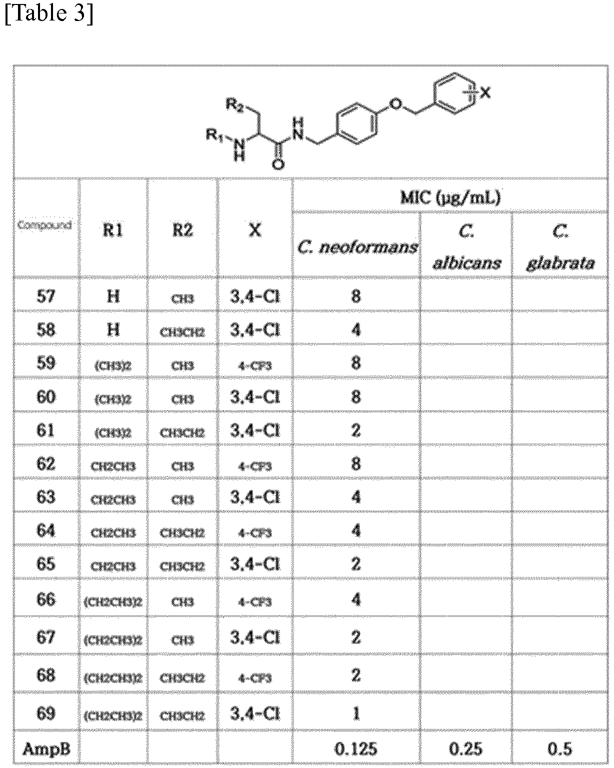

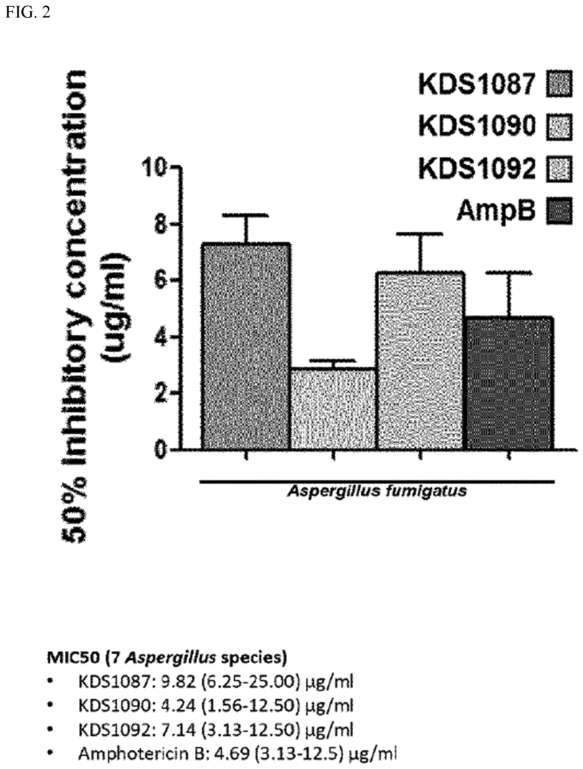

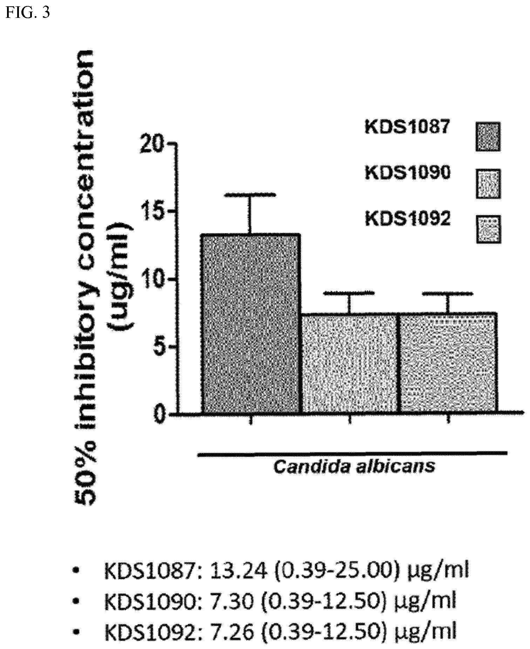

FIG. 2 illustrates a graph obtained by comparing MIC test results of Compound 22 (1087), Compound 26 (1090), and Compound 28 (1092) of the present invention with that of amphotericin B (AMB), against several types of *Aspergillus fumigatus* strains (KCCM32791, KCCM60031, KCCM60293, KCCM60036, KCCM60031, and KCCM60027) (average values are indicated). The compound of the present invention exhibited a higher inhibitory activity than amphotericin B. FIG. 3 illustrates average values of MIC test results of Compound 22 (1087), Compound 26 (1090), and Compound 28 (1092) of the present invention, against several types of *Candida albicans* strains (KCCM11282, KCCM11474, KCCM12552, KCCM12554, KCCM50235, KCCM50539, KCCM 50582, KCCM50573, and KCCM12556). In addition, in a case where compound of the present invention is applied to *Cryptococcus neoformans*, *Candida glabrata*, *Candida albicans*, and the like, the compound of the present invention has a synergistic effect at the time of being administered in combination with itraconazole and voriconazole which are commercially available as antifungal agents (results not shown). This suggests the possibility of combined administration with resistant drugs, in particular, azole-based drugs.

### Test Example 2: Suppressive activity against phytopathogenic fungi and Trichophytons

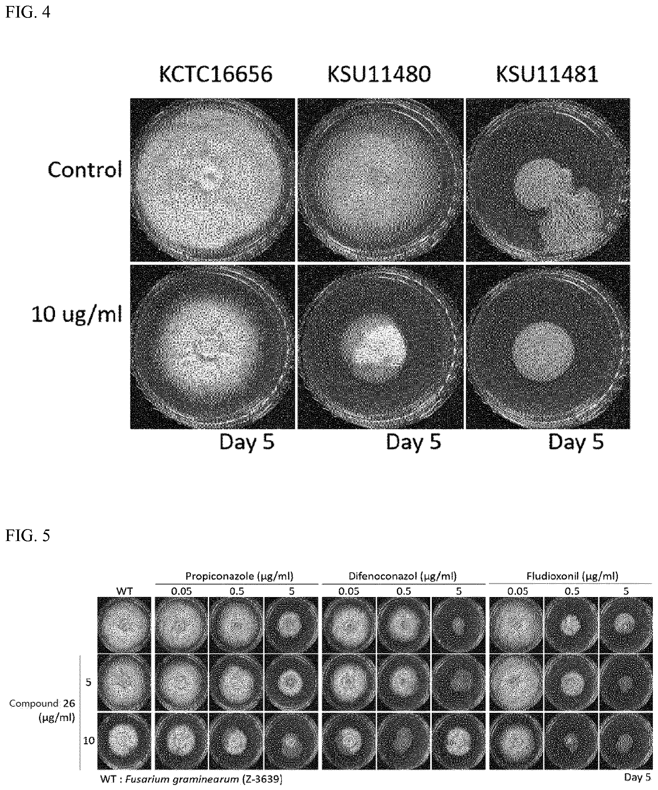

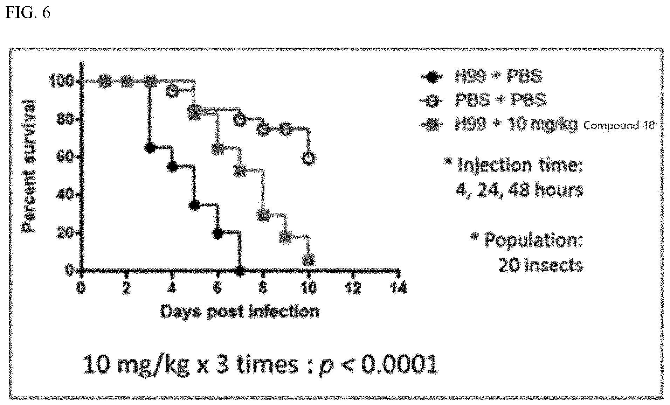

*Fusarium graminearum* (KCTC16656) and *Fusarium* (KSU11480, strain that produces fumonisin which is a cereal toxin), which are phytopathogenic fungi, were cultured in a YPD solid medium (Sigma-Aldrich), and the compound of the present invention (Compound 26) was applied thereto. As a result, it was identified that the compound has a very excellent suppressive activity. Plating the pathogenic fungi on a solid medium and performing culture was carried out using a generally known and established method. The results are shown in FIG. 4. FIG. 5 illustrates results obtained by comparing suppresive activity of the compound of the present invention with those of propiconazole, difenoconazole, and fludioxonil. From this, it was identified that the compound of the present invention has a similar level of activity to these agents. FIG. 6 illustrates results obtained by culturing strains *Trichophyton tonsurans* (KCCM60442), *Trichophyton tonsurans* (KCCM11866), *Trichophyton rubrum* (KCCM60450), *Trichophyton mentagrophytes* (KCCM11950), *Trichophyton mentagrophytes* (KCCM60449), and *Trichophyton mentagrophytes* (KCCM60444) in a YPD solid medium, applying the compound of the present invention (Compound 26) thereto, and observing suppressive activity thereof. As identified in FIG. 6, the compound of the present invention also exhibited excellent suppressive activity against these Trichophytons.

### Test Example 3: Assessment of drug efficacy using Galleria mellonella insect model (in vivo)

The wild-type (H99S) strain of *Cryptococcus neoformans,* which is pathogenic fungi, was inoculated into a yeast extract, peptone, and dextrose (YPD) liquid medium, cultured for 16 hours, and then washed twice with phosphate buffered saline (PBS), to prepare a final sample of 10⁶ cells/ml. As the insect model, *Galleria mellonella*, 7-day-old or younger larvae, which had been delivered from the manufacturer (Vanderhorst, U.S.A.), were used, and 20 individuals that weigh about 200 to 300 mg were prepared per experimental drug group. A 4 µl suspension of *C. neoformans* prepared was injected into a proleg of larvae, so that 4,000 pathogen cells were infected per larva, in which an automatic injector (PB600-1) manufactured by Hamilton Company, and 100 µl syringes and needles were used. 3 hours after the pathogen infection, each drug (Compound 18 of the present invention) diluent was injected in the same manner, and then the drug was additionally injected for two days, once every 24 hours. A group into which PBS alone was injected each time after the infection with *C. neoformans* was tested as a positive control, and a group in which both infection and drug injection were replaced with PBS was tested as a negative control. After the infection and the drug injection, the larvae were observed daily in an incubator at 37°C in an environment where sufficient humidity could be maintained, and a survival rate was recorded. The larvae were determined as dead in a case where the larvae became dark-colored and made no movement. A chart depicting survival rates was prepared using the GraphPad Prism6 program. For significance with the controls, a significance level was determined using the Long-rank (Mantel-Cox) method.

As illustrated in FIG. 6, for the larva group fed with PBS alone after the fungal infection, the survival rate thereof dropped to 60% on day 3 of the infection, and all died on day 7 of the infection, whereas for the larva group fed with the drug according to the present invention, all survived on day 3 of the infection and 50% survival rate was exhibited on day 7 of the infection. Therefore, it was identified that the compound of the present invention exhibits an antifungal effect even in an animal model, and has very low toxicity.

The novel compound of the present invention is a compound having an excellent antifungal effect and can be usefully used for the development of antifungal agents. In addition, the compound has broad fungicidal activity and can be used for the prevention and treatment of human infectious fungi as well as for the development of antifungal or fungicidal preparations for preventing and killing death of, animal infectious fungal diseases and phytopathogenic fungi. In addition, the compound may also be used for the development of preparations for combination administration, since the compound exhibits a synergistic effect in a case of being used together with an existing antifungal agent.

## Claims

1. A benzyloxybenzylamine amino acid derivative represented by the following Formula 1, a salt and/or a solvate thereof: 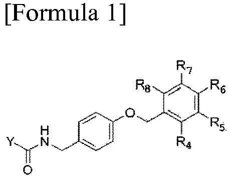 in the Formula 1,
R₄, R₅, R₆, R₇, and R₈ are the same or different from one another, and are each independently selected from hydrogen, C₁₋₇ alkyl, hydroxy, halogen, halogenated C₁₋₇ alkyl, C₁₋₇ alkyloxy, and halogenated C₁₋₇ alkyloxy, and
Y is selected from the following Formulas 2 to 4, 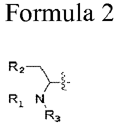 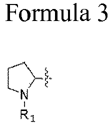 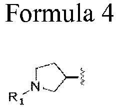 where R₁ and R₃ are the same or different from each other, and are each independently hydrogen or C₁₋₇ alkyl, and
R₂ is C₁₋₇ alkyl.

2. The benzyloxybenzylamine amino acid derivative, a salt and/or a solvate thereof according to claim 1,
wherein the C₁₋₇ alkyl is linear, branched, or cyclic C₁₋₇ alkyl.

3. The benzyloxybenzylamine amino acid derivative, a salt and/or a solvate thereof according to claim 1,
wherein the compound represented by the Formula 1 is any one selected from the group consisting of the following compounds, or a racemic mixture of R-form and S-form thereof:
*(R)*/*(S)*-1-((4-((2-(fluorobenzyl)oxy)benzyl)amino)-1-oxobutane-2-aminium chloride (Compound 17);
*(R)*/*(S)*-1-((4-((3-(fluorobenzyl)oxy)benzyl)amino)-1-oxobutane-2-aminium chloride (Compound 18);
*(R)*/*(S)*-1-((4-((4-(fluorobenzyl)oxy)benzyl)amino)-1-oxobutane-2-aminium chloride (Compound 19);
*(R)*/*(S*)-1-oxo-1-((4-((2-(trifluoromethyl)benzyl)oxy)benzyl)amino)butane-2-aminium chloride (Compound 20);
*(R)*/*(S*)-1-oxo-1-((4-((3-(trifluoromethyl)benzyl)oxy)benzyl)amino)butane-2-aminium chloride (Compound 21);
*(R)*/*(S*)-1-oxo-1-((4-((4-(trifluoromethyl)benzyl)oxy)benzyl)amino)butane-2-aminium chloride (Compound 22);
*(R)*/*(S)*-1-((4-((3-(chlorobenzyl)oxy)benzyl)amino)-1-oxobutane-2-aminium chloride (Compound 23);
*(R)*/*(S)*-1-((4-((4-(chlorobenzyl)oxy)benzyl)amino)-1-oxobutane-2-aminium chloride (Compound 24);
*(R)*/*(S)-*1-((4-((3-fluorobenzyl)oxy)benzyl)amino)-1-oxopentane-2-aminium chloride (Compound 25);
*(R)*/*(S*)-1-((4-((4-(trifluorobenzyl)oxy)benzyl)amino)-1-oxopentane-2-aminium chloride (Compound 26);
*(R)*/*(S*)-2-(methylamino)-N-(4-((4-trifluoromethyl)benzyl)oxy)benzyl)butanamide (Compound 27);
*(R)*/*(S*)-N-(4-((3-chlorobenzyl)oxy)benzyl)-2-(methylamino)butanamide (Compound 28);
*(R)*/*(S*)-N-(4-((4-chlorobenzyl)oxy)benzyl)-2-(methylamino)butanamide (Compound 29);
*(R)*/*(S*)-2-(methylamino)-N-(4-((4-trifluoromethyl)benzyl)oxy)benzyl)pentanamide (Compound 30);
*(R)*/*(S*)-2-(methylamino)-N-(4-((4-trifluoromethyl)benzyl)oxy)benzyl)pentanamide (Compound 31);
*(R)*/*(S)*-tert-butyl-2-((4-((3-fluorobenzyl)oxy)benzyl)carbamoyl)pyrrolidine-1-carboxylate (Compound 34);
(*R*)/(*S*)-tert-butyl-2-((4-((4-fluorobenzyl)oxy)benzyl)carbamoyl)pyrrolidine-1-carboxylate (Compound 35);
(*R*)/(*S*)-tert-butyl-2-((4-((4-(trifluoromethyl)benzyl)oxy)benzyl)carbamoyl)pyrrolidine-1-carboxylate (Compound 36);
(*R*)/(*S*)-tert-butyl-2-((4-((4-chlorobenzyl)oxy)benzyl)carbamoyl)pyrrolidine-1-carboxylate (Compound 37);
(*R*)/(*S*)-N-(4-((3-fluorobenzyl)oxy)benzyl)pyrrolidine-2-carboxamide hydrochloride (Compound 38)
*(R)*/*(S*)-N-(4-((4-fluorobenzyl)oxy)benzyl)pyrrolidine-2-carboxamide hydrochloride (Compound 39);
*(R)*/*(S*)-N-(4-((4-(trifluoromethyl)benzyl)oxy)benzyl)pyrrolidine-2-carboxamide hydrochloride (Compound 40);
*(R)*/*(S*)-N-(4-((4-chlorobenzyl)oxy)benzyl)pyrrolidine-2-carboxamide hydrochloride (Compound 41);
*(R)*/*(S*)-1-methyl-N-(4-((3-fluorobenzyl)oxy)benzyl)pyrrolidine-2-carboxamide (Compound 42);
*(R)*/*(S*)-1-methyl-N-(4-((4-(trifluoromethyl)benzyl)oxy)benzyl)pyrrolidine-2-carboxamide (Compound 43);
*(R)*/*(S*)-1-methyl-N-(4-((4-chlorobenzyl)oxy)benzyl)pyrrolidine-2-carboxamide (Compound 44);
*(R)*/*(S*)-tert-butyl-3-((4((3-fluorobenzyl)oxy)benzyl)carbamoyl)pyrrolidine-1-carboxylate (Compound 46);
*(R)*/*(S*)-tert-butyl-3-((4((4-(trifluoromethyl)benzyl)oxy)benzyl)carbamoyl)pyrrolidine-1-carboxylate (Compound 47);
*(R)*/*(S*)-tert-butyl-3-((4((4-chlorobenzyl)oxy)benzyl)carbamoyl)pyrrolidine-1-carboxylate (Compound 48);
*(R)*/*(S*)-2-((4-((3-fluorobenzyl)oxy)benzyl)carbamoyl)pyrrolidi-1-nium chloride (Compound 49);
*(R)*/*(S*)-2-((4-((4-(trifluoromethyl)benzyl)oxy)benzyl)carbamoyl)pyrrolidi-1-nium chloride (Compound 50);
*(R)*/*(S*)-2-((4-((4-chlorobenzyl)oxy)benzyl)carbamoyl)pyrrolidi-1-nium chloride (Compound 51);
*(R)*/*(S*)-1-methyl-N-(4-((3-fluorobenzyl)oxy)benzyl)pyrrolidine-3-carboxamide (Compound 52);
*(R)*/*(S*)-1-methyl-N-(4-((4-(trifluoromethyl)benzyl)oxy)benzyl)pyrrolidine-3-carboxamide (Compound 53);
*(R)*/*(S*)-1-methyl-N-(4-((4-chlorobenzyl)oxy)benzyl)pyrrolidine-3-carboxamide (Compound 54);
*(R)*/*(S*)-tert-butyl(1-((4-((3,4-chlorobenzyl)oxy)benzyl)amino)-1-oxobuta-2-nyl)carbamate (Compound 55);
*(R)*/*(S*)-tert-butyl(1-oxo-1-((4-((4-(trifluoromethyl)benzyl)oxy)benzyl)amino)penta-2-nyl)carbamate (Compound 56);
*(R)*/*(S)*-1-((4-((3,4-dichlorobenzyl)oxy)benzyl)amino)-1-oxobutane-2-aminium chloride (Compound 57);
*(R)*/*(S)*-1-((4-((3,4-dichlorobenzyl)oxy)benzyl)amino)-1-oxopentane-2-aminium chloride (Compound 58);
*(R)*/*(S*)-2-(dimethylamino)-N-(4-((4-trifluoromethyl)benzyl)oxy)benzyl)butanamide (Compound 59);
*(R)*/*(S*)-N-(4-((3,4-dichlorobenzyl)oxy)benzyl)-2-(dimethylamino)butanamide (Compound 60);
*(R)*/*(S*)-N-(4-((3,4-dichlorobenzyl)oxy)benzyl)-2-(dimethylamino)butanamide (Compound 61);
*(R)*/*(S*)-2-(ethylamino)-N-(4-((4-(trifluoromethyl)benzyl)oxy)benzyl)butanamide (Compound 62);
*(R)*/*(S*)-N-(4-((3,4-dichlorobenzyl)oxy)benzyl-2-(ethylamino)butanamide (Compound 63);
*(R)*/*(S*)-2-(ethylamino)-N-(4-((4-(trifluoromethyl)benzyl)oxy)benzyl)pentanamide (Compound 64);
*(R)*/*(S*)-N-(4-((3,4-dichlorobenzyl)oxy)benzyl-2-(ethylamino)pentanamide (Compound 65);
*(R)*/*(S*)-2-(diethylamino)-N-(4-((4-(trifluoromethyl)benzyl)oxy)benzyl)butanamide (Compound 66);
*(R)*/*(S*)-N-(4-((3,4-dichlorobenzyl)oxy)benzyl-2-(diethylamino)butanamide (Compound 67);
*(R)*/*(S*)-2-(diethylamino)-N-(4-((4-(trifluoromethyl)benzyl)oxy)benzyl)pentanamide (Compound 68); and
*(R)*/*(S*)-N-(4-((3,4-dichlorobenzyl)oxy)benzyl-2-(diethylamino)pentanamide (Compound 69).

4. A pharmaceutical composition for preventing or treating mycosis, comprising as an active ingredient:
a benzyloxybenzylamine amino acid derivative represented by the following Formula 1 or a pharmaceutically acceptable salt thereof: 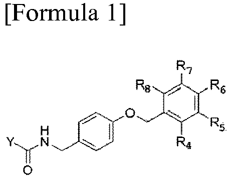 in the Formula 1,
R₄, R₅, R₆, R₇, and R₈ are the same or different from one another, and are each independently selected from hydrogen, C₁₋₇ alkyl, hydroxy, halogen, halogenated C₁₋₇ alkyl, C₁₋₇ alkyloxy, and halogenated C₁₋₇ alkyloxy, and
Y is selected from the following Formulas 2 to 4, 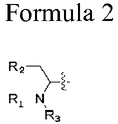 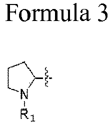 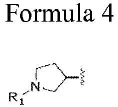 where R₁ and R₃ are the same or different from each other, and are each independently hydrogen or C₁₋₇ alkyl, and
R₂ is C₁₋₇ alkyl.

5. The pharmaceutical composition according to claim 4, which has antimicrobial activity against fungi selected from Candida *spp.*, Sporotrichum *spp.*, Malassezia *spp.*, Cryptococcus *spp.*, Aspergillus *spp.*, Pneumocystis jirovecii *spp.*, and Mucor *spp.*

6. The pharmaceutical composition according to claim 4,
wherein the mycosis is selected from encephalomeningitis, encephalitis caused by encephalomeningitis, skin candidiasis, mucosal candidiasis, visceral candidiasis, urinary candidiasis, candida endocarditis, oropharyngeal candidiasis, candida endophthalmitis, candida septicemia, trichophytosis, tinea versicolor, sporotrichosis, fungal abscess, fungal granuloma, granuloma pyogenicum, maduromycosis, pneumocystis jirovecii pneumonia, systemic cryptococcosis, skin mucosal cryptococcosis, aspergillosis, cavity, hemoptysis, allergy, old pulmonary tuberculosis, pulmonary fibrosis, pulmonary cyst, chronic fever, cough, sputum, blood-stained sputum, tinea cruris, thrush, fungal infection-induced dementia, and zygomycosis.

7. The pharmaceutical composition according to claim 4,
wherein the compound represented by the Formula 1 is any one selected from the group consisting of the following compounds, or a racemic mixture of R-form and S-form thereof:
*(R)*/*(S)*-1-((4-((2-(fluorobenzyl)oxy)benzyl)amino)-1-oxobutane-2-aminium chloride (Compound 17);
*(R)*/*(S)*-1-((4-((3-(fluorobenzyl)oxy)benzyl)amino)-1-oxobutane-2-aminium chloride (Compound 18);
*(R)*/*(S)*-1-((4-((4-(fluorobenzyl)oxy)benzyl)amino)-1-oxobutane-2-aminium chloride (Compound 19);
*(R)*/*(S)*-1-oxo-1-((4-((2-(trifluoromethyl)benzyl)oxy)benzyl)amino)butane-2-aminium chloride (Compound 20);
*(R)*/*(S)*-1-oxo-1-((4-((3-(trifluoromethyl)benzyl)oxy)benzyl)amino)butane-2-aminium chloride (Compound 21);
*(R)*/*(S)*-1-oxo-1-((4-((4-(trifluoromethyl)benzyl)oxy)benzyl)amino)butane-2-aminium chloride (Compound 22);
*(R)*/*(S)*-1-((4-((3-(chlorobenzyl)oxy)benzyl)amino)-1-oxobutane-2-aminium chloride (Compound 23);
*(R)*/*(S)*-1-((4-((4-(chlorobenzyl)oxy)benzyl)amino)-1-oxobutane-2-aminium chloride (Compound 24);
*(R)*/*(S)-*1-((4-((3-fluorobenzyl)oxy)benzyl)amino)-1-oxopentane-2-aminium chloride (Compound 25);
*(R)*/*(S)*-1-((4-((4-(trifluorobenzyl)oxy)benzyl)amino)-1-oxopentane-2-aminium chloride (Compound 26);
*(R)*/*(S)*-2-(methylamino)-N-(4-((4-trifluoromethyl)benzyl)oxy)benzyl)butanamide (Compound 27);
*(R)*/*(S*)-N-(4-((3-chlorobenzyl)oxy)benzyl)-2-(methylamino)butanamide (Compound 28);
*(R)*/*(S*)-N-(4-((4-chlorobenzyl)oxy)benzyl)-2-(methylamino)butanamide (Compound 29);
*(R)*/*(S*)-2-(methylamino)-N-(4-((4-trifluoromethyl)benzyl)oxy)benzyl)pentanamide (Compound 30);
*(R)*/*(S*)-2-(methylamino)-N-(4-((4-trifluoromethyl)benzyl)oxy)benzyl)pentanamide (Compound 31);
*(R)*/*(S*)-tert-butyl-2-((4-((3-fluorobenzyl)oxy)benzyl)carbamoyl)pyrrolidine-1-carboxylate (Compound 34);
*(R)*/*(S*)-tert-butyl-2-((4-((4-fluorobenzyl)oxy)benzyl)carbamoyl)pyrrolidine-1-carboxylate (Compound 35);
*(R)*/*(S*)-tert-butyl-2-((4-((4-(trifluoromethyl)benzyl)oxy)benzyl)carbamoyl)pyrrolidine-1-carboxylate (Compound 36);
*(R)*/*(S*)-tert-butyl-2-((4-((4-chlorobenzyl)oxy)benzyl)carbamoyl)pyrrolidine-1-carboxylate (Compound 37);
*(R)*/*(S*)-N-(4-((3-fluorobenzyl)oxy)benzyl)pyrrolidine-2-carboxamide hydrochloride (Compound 38) *(R)*/*(S*)*-*N-(4-((4-fluorobenzyl)oxy)benzyl)pyrrolidine-2-carboxamide hydrochloride (Compound 39);
*(R)*/*(S*)-N-(4-((4-(trifluoromethyl)benzyl)oxy)benzyl)pyrrolidine-2-carboxamide hydrochloride (Compound 40);
*(R)*/*(S*)-N-(4-((4-chlorobenzyl)oxy)benzyl)pyrrolidine-2-carboxamide hydrochloride (Compound 41);
*(R)*/*(S*)-1-methyl-N-(4-((3-fluorobenzyl)oxy)benzyl)pyrrolidine-2-carboxamide (Compound 42);
*(R)*/*(S*)-1-methyl-N-(4-((4-(trifluoromethyl)benzyl)oxy)benzyl)pyrrolidine-2-carboxamide (Compound 43);
*(R)*/*(S*)-1-methyl-N-(4-((4-chlorobenzyl)oxy)benzyl)pyrrolidine-2-carboxamide (Compound 44);
*(R)*/*(S*)-tert-butyl-3-((4((3-fluorobenzyl)oxy)benzyl)carbamoyl)pyrrolidine-1-carboxylate (Compound 46);
*(R)*/*(S*)-tert-butyl-3-((4((4-(trifluoromethyl)benzyl)oxy)benzyl)carbamoyl)pyrrolidine-1-carboxylate (Compound 47);
*(R)*/*(S*)-tert-butyl-3-((4((4-chlorobenzyl)oxy)benzyl)carbamoyl)pyrrolidine-1-carboxylate (Compound 48);
*(R)*/*(S*)-2-((4-((3-fluorobenzyl)oxy)benzyl)carbamoyl)pyrrolidi-1-nium chloride (Compound 49);
*(R)*/*(S*)-2-((4-((4-(trifluoromethyl)benzyl)oxy)benzyl)carbamoyl)pyrrolidi-1-nium chloride (Compound 50);
*(R)*/*(S*)-2-((4-((4-chlorobenzyl)oxy)benzyl)carbamoyl)pyrrolidi-1-nium chloride (Compound 51);
*(R)*/*(S*)-1-methyl-N-(4-((3-fluorobenzyl)oxy)benzyl)pyrrolidine-3-carboxamide (Compound 52);
*(R)*/*(S*)-1-methyl-N-(4-((4-(trifluoromethyl)benzyl)oxy)benzyl)pyrrolidine-3-carboxamide (Compound 53);
*(R)*/*(S*)-1-methyl-N-(4-((4-chlorobenzyl)oxy)benzyl)pyrrolidine-3-carboxamide (Compound 54);
*(R)*/*(S*)-tert-butyl(1-((4-((3,4-chlorobenzyl)oxy)benzyl)amino)-1-oxobuta-2-nyl)carbamate (Compound 55);
*(R)*/*(S*)*-*tert-butyl(1-oxo-1-((4-((4-(trifluoromethyl)benzyl)oxy)benzyl)amino)penta-2-nyl)carbamate (Compound 56);
*(R)*/*(S*)-1-((4-((3,4-dichlorobenzyl)oxy)benzyl)amino)-1-oxobutane-2-aminium chloride (Compound 57);
*(R)*/*(S*)-1-((4-((3,4-dichlorobenzyl)oxy)benzyl)amino)-1-oxopentane-2-aminium chloride (Compound 58);
*(R)*/*(S*)-2-(dimethylamino)-N-(4-((4-trifluoromethyl)benzyl)oxy)benzyl)butanamide (Compound 59);
*(R)*/*(S*)-N-(4-((3,4-dichlorobenzyl)oxy)benzyl)-2-(dimethylamino)butanamide (Compound 60);
*(R)*/*(S*)-N-(4-((3,4-dichlorobenzyl)oxy)benzyl)-2-(dimethylamino)butanamide (Compound 61);
*(R)*/*(S*)-2-(ethylamino)-N-(4-((4-(trifluoromethyl)benzyl)oxy)benzyl)butanamide (Compound 62);
*(R)*/*(S*)-N-(4-((3,4-dichlorobenzyl)oxy)benzyl-2-(ethylamino)butanamide (Compound 63);
*(R)*/*(S*)-2-(ethylamino)-N-(4-((4-(trifluoromethyl)benzyl)oxy)benzyl)pentanamide (Compound 64);
*(R)*/*(S*)-N-(4-((3,4-dichlorobenzyl)oxy)benzyl-2-(ethylamino)pentanamide (Compound 65);
*(R)*/*(S*)-2-(diethylamino)-N-(4-((4-(trifluoromethyl)benzyl)oxy)benzyl)butanamide (Compound 66);
*(R)*/*(S*)-N-(4-((3,4-dichlorobenzyl)oxy)benzyl-2-(diethylamino)butanamide (Compound 67);
*(R)*/*(S*)-2-(diethylamino)-N-(4-((4-(trifluoromethyl)benzyl)oxy)benzyl)pentanamide (Compound 68); and
*(R)*/*(S*)-N-(4-((3,4-dichlorobenzyl)oxy)benzyl-2-(diethylamino)pentanamide (Compound 69).

8. A method for preparing a benzyloxybenzylamine amino acid derivative represented by the following Formula 1, comprising the steps of:
i) protecting an amino group of an amino acid selected from the following Formulas 5 to 7 with a protecting group;
ii) reacting the amino acid having a protected amine group with a benzylamine derivative, to prepare a benzyloxybenzylamine amino acid derivative in which the amino group of the amino acid is protected with the protecting group; and
iii) removing the amine protecting group, 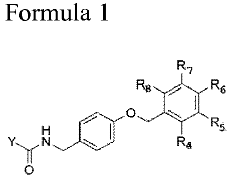 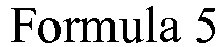 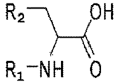 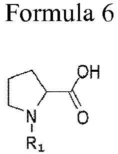 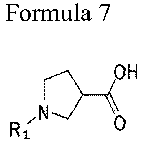 in the Formula 1,
R₄, R₅, R₆, R₇, and R₈ are the same or different from one another, and are each independently selected from hydrogen, C₁₋₇ alkyl, hydroxy, halogen, halogenated C₁₋₇ alkyl, C₁₋₇ alkyloxy, and halogenated C₁₋₇ alkyloxy, and
Y is selected from the following Formulas 2 to 4, 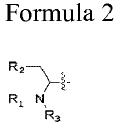 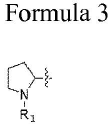 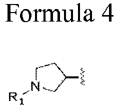 in the Formulas 2 to 7,
R₁ and R₃ are the same or different from each other, and are each independently hydrogen or C₁₋₇ alkyl, and
R₂ is C₁₋₇ alkyl.

9. The method according to claim 8,
wherein the step (ii) includes reacting the amino acid having a protected amine group with a benzyloxybenzylamine derivative.

10. The method according to claim 8,
wherein the step ii) includes reacting the amino acid having a protected amine group with 4-hydroxybenzylamine to prepare a 4-hydroxybenzylamine amino acid derivative, in which the amine group of the amino acid is protected with a protecting group, and then reacting the resultant with a benzyl bromide derivative.

11. The method according to claim 8,
wherein step iii) further includes a step of performing alkylation after removing the amine protecting group.

12. The method according to claim 8,
wherein the compound represented by Formula 1 has anti-microbial activity against fungi selected from Candida *spp*., Sporotrichum *spp*., Malassezia *spp.,* Cryptococcus *spp*., Aspergillus *spp*., Pneumocystis jirovecii *spp*., and Mucor *spp*.

13. A pharmaceutical composition for preventing or treating an animal pathogenic fungal infection, comprising as an active ingredient:
a benzyloxybenzylamine amino acid derivative represented by the following Formula 1 or a pharmaceutically acceptable salt thereof: 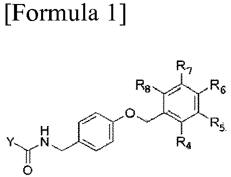 in the Formula 1,
R₄, R₅, R₆, R₇, and R₈ are the same or different from one another, and are each independently selected from hydrogen, C₁₋₇ alkyl, hydroxy, halogen, halogenated C₁₋₇ alkyl, C₁₋₇ alkyloxy, and halogenated C₁₋₇ alkyloxy, and
Y is selected from the following Formulas 2 to 4, 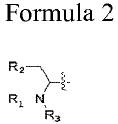 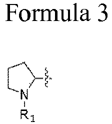 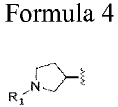 where R₁ and R₃ are the same or different from each other, and are each independently hydrogen or C₁₋₇ alkyl, and
R₂ is C₁₋₇ alkyl.

14. A pharmaceutical composition for preventing or treating a phytopathogenic fungal infection, comprising as an active ingredient:
a benzyloxybenzylamine amino acid derivative represented by the following Formula 1 or a pharmaceutically acceptable salt thereof: 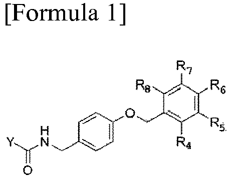 in the Formula 1,
R₄, R₅, R₆, R₇, and R₈ are the same or different from one another, and are each independently selected from hydrogen, C₁₋₇ alkyl, hydroxy, halogen, halogenated C₁₋₇ alkyl, C₁₋₇ alkyloxy, and halogenated C₁₋₇ alkyloxy, and
Y is selected from the following Formulas 2 to 4, 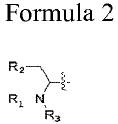 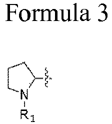 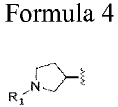 where R₁ and R₃ are the same or different from each other, and are each independently hydrogen or C₁₋₇ alkyl, and
R₂ is C₁₋₇ alkyl.

15. A cosmetic composition or human body cleansing composition, comprising as an active ingredient:
a benzyloxybenzylamine amino acid derivative represented by the following Formula 1 or a pharmaceutically acceptable salt thereof: 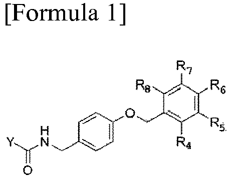 in the Formula 1,
R₄, R₅, R₆, R₇, and R₈ are the same or different from one another, and are each independently selected from hydrogen, C₁₋₇ alkyl, hydroxy, halogen, halogenated C₁₋₇ alkyl, C₁₋₇ alkyloxy, and halogenated C₁₋₇ alkyloxy, and
Y is selected from the following Formulas 2 to 4, 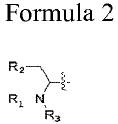 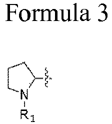 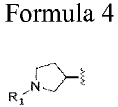 where R₁ and R₃ are the same or different from each other, and are each independently hydrogen or C₁₋₇ alkyl, and
R₂ is C₁₋₇ alkyl.
